# EUROPEAN PATENT APPLICATION

(11) **EP 4 527 932 A1**
(43) Date of publication of application: **26.03.2025**
(21) Application number: 23807054.4
(22) Date of filing: 19.05.2023
(51) Int. Cl.: C12N 15/85, C12N 15/63, C12N 15/11, A61K 38/00, A61K 39/00, C07K 14/00

(54) **NUCLEIC ACID CONSTRUCT FOR ACHIEVING MODULAR LOADING OF ENGINEERED EVS FUNCTIONAL PROTEIN AND USE OF SAID CONSTRUCT**

(30) Priority: 20.05.2022 CN 202210549553
(71) Applicant: Panexo Biotech Sg Pte.Ltd, Singapore 409051 (SG)
(72) Inventor: WANG, Yi, Beijing 100141 (CN); SONG, Haifeng, Beijing 100141 (CN); DONG, Yanan, Beijing 100141 (CN); XUE, Miaomiao, Beijing 100141 (CN); LI, Yanfang, Beijing 100141 (CN); ZHANG, Jing, Beijing 100141 (CN); ZHOU, Wei, Beijing 100141 (CN); XUE, Lijia, Beijing 100141 (CN)
(74) Representative: Lavoix
(86) International application number: PCT/CN2023/095183
(87) International publication number: WO 2023/222105

(57) **Abstract**

Provided are a nucleic acid construct for achieving modular loading of an engineered EVs functional protein and use of said construct. A modular design principle is adopted, specific modules are selected for combination, a mutant sequence is optimized and screened to obtain an improved nucleic acid construct, and finally, the engineered EVs modularly loaded with functional protein is converted and expressed. The product has the following advantages that: I) the modular loading of the engineered EVs functional protein is achieved; 2) the loading density and the loading efficiency of the protein inside and outside an EVs membrane are improved; and 3) a specific support sequence module is selected to avoid enzyme digestion. The construct is used to prepare the engineered EVs, so that the modular loading of the functional protein can be achieved, the loading efficiency is improved, and the construct has a wide clinical application value and market prospect.

## Description

This application claims the priority of Chinese Patent Application No. 202210549553.7, filed with the China National Intellectual Property Administration on May 20, 2022, and titled with "NUCLEIC ACID CONSTRUCT FOR ACHIEVING MODULAR LOADING OF ENGINEERED EVs FUNCTIONAL PROTEIN AND USE OF SAID CONSTRUCT", the disclosure of which is hereby incorporated by reference in its entirety.

### FIELD

The present disclosure relates to the technical field of extracellular vesicles (EVs) and use thereof, in particular to a nucleic acid construct for modular loading of a functional protein into engineered EVs and use thereof.

### BACKGROUND

Extracellular vehicles (EVs) are primarily classified into exosomes, microvesicles (MVs) or microparticles (MPs), and apoptotic bodies based on their size and origin. Exosomes are vesicles formed by the fusion of multivesicular bodies (MVBs) with the plasma membrane, MVs are formed by direct budding from the plasma membrane, and apoptotic bodies are released after cell apoptosis. EVs are secreted by all types of cells in vivo and are useful in intercellular communication. Depending on their size, EVs include small vesicles (10-100 nm) from multivesicular bodies, microvesicles (100-1000 nm) from the plasma membrane of living cells, and apoptotic bodies (500-5000 nm) from the plasma membrane of apoptotic cells. In nanomedicine, most research focus on exosomes and MVs/MPs, with less emphasis on apoptotic bodies. Extracellular vesicles are membrane-derived vesicles, and thus they contain a membrane, typically a lipid bilayer.

Over the past few decades, the biological functions of EVs have been widely reported. EVs are nanoscale vesicles that can transfer bioactive molecules from donor cells to recipient cells through various mechanisms, including membrane fusion, receptor-ligand interaction, endocytosis, or phagocytosis. EVs are emerging as novel drug delivery carriers due to their natural biocompatibility, high delivery efficiency, low toxicity, and low immunogenicity. Compared to conventional nanomaterials, EVs offer advantages such as biocompatibility, biodegradability, low toxicity, and non-immunogenicity, making them one of the most promising candidates in nanomedicine. EVs resemble liposomes in size, shape, and structure but feature a more complex bilayer structure that includes hundreds of different types of lipids, proteins, carbohydrates, internal cargo, and surface molecules. EVs play a crucial role in various pathophysiological processes and in both short-distance and long-distance intercellular communication. Their ability to transport biomolecules to recipient cells makes them attractive for drug delivery.

EVs can be obtained from conditioned media for culturing cells or from biological tissues or fluids, and various methods (e.g., electroporation, extrusion, and sonication) have been used to load therapeutic agents into EVs. Typically, EVs are engineered by genetic modification of their donor cells. Drug (including small molecules, proteins, and nucleic acids) delivery via EVs is an appealing platform due to the natural biocompatibility of EVs, which overcomes most in vivo delivery barriers. EVs are generally non-toxic and non-immunogenic. They are readily taken up by many cell types, although they may possess some anti-phagocytic markers (e.g., CD47) that help them evade phagocytosis by macrophages and monocytes in the reticuloendothelial system. Moreover, EVs can efficiently permeate through endothelial junctions and even cross the blood-brain barrier, making them versatile drug carriers.

EVs are derived from various tissues or cells, exhibit low cytotoxicity, and are stable with low immunogenicity, even capable of crossing the blood-brain barrier. They also offer advantages in protecting and stabilizing drug activity. Cell membranes and cell-derived EVs enable carriers to effectively cross biological barriers and target tumor tissues. Some cells can be used to extract membranes for preparing carriers, while EVs produced by the cells can be used for drug delivery. Although membrane extraction and preparation are relatively easy, some proteins may be lost during membrane extraction, reducing targeting ability to a certain extent. In contrast, EVs, while more challenging to prepare, can preserve membrane components more completely and demonstrate excellent targeting ability. EVs have been employed to load a range of drugs, including small molecules, proteins, and nucleic acids. The loaded protein is usually overexpressed, and the engineered exosomes loaded with the protein are extracted.

Existing engineering loading technologies involve fusing the expression protein with a membrane localization element in exosome donor cells, and loading the protein onto the exosome membrane via the membrane localization element. The membrane localization element usually utilizes transmembrane elements from EV surface biomarkers like CD9 and CD81 to construct plasmids that contain fusion proteins and transmembrane elements. These plasmids are then overexpressed in cells, and the resulting engineered EVs are purified, though the loading density remains limited. Scaffold sequences using EWI-F and EWI-2 are susceptible to cleavage by ADAM10. Therefore, there is a need to develop a nucleic acid construct that protect against enzymatic cleavage and enable efficient loading of engineered EVs.

### SUMMARY

In order to address the deficiencies in the prior art, the present disclosure provides a nucleic acid construct for modular loading of a functional protein into engineered EVs, and use thereof. This method enables the modular loading of a functional protein into engineered EVs, thereby enhancing the efficiency of protein loading both inside and outside the EV membrane.

In order to achieve the above objects and to solve the problem, in a first aspect, the present disclosure provides a nucleic acid construct for modular loading of a functional protein into engineered EVs, comprising:
an effector module (Head), a scaffold module (Scaffold), a transmembrane domain module (TMD), and an intracellular domain anchoring module (ICD);
wherein the effector module, the scaffold module, the transmembrane domain module and the intracellular domain anchoring module are sequentially connected from 5' end to 3' end either directly or through a linking module, and the linking module comprises a linker peptide (Linker).

The linker peptide is classified into rigid and flexible linker peptides. Common sequences of rigid linker peptides include (EAAAK)ₙ (n=1-3) and common sequences of flexible linker peptides include (GGGGS)ₙ (n=1-5).

Preferably, the nucleic acid construct further comprises a detection module (Tail), wherein the 5' end of the detection module is connected to the 3' end of the intracellular domain anchoring module.

Preferably, a flexible linker peptide is used between the modules of the present disclosure.

Preferably, the liner peptide of the present disclosure has an amino acid sequence of (GGGGS)₃.

Preferably, the nucleic acid construct designed in the present disclosure comprises the following modules:
Head-Scaffold-TMD-ICD-Tail.

Head represents the effector protein loaded on the extracellular side of the membrane. Scaffold represents the scaffold protein on the extracellular side, connected to the C-terminal end of the effector protein. TMD represents the transmembrane domain, with the N- and C-terminal ends connected to the extracellular and intracellular sequences of the EV membrane, respectively. ICD represents the intracellular domain of the transmembrane proteins, containing S-palmitoyl cysteine for anchoring to the EVs membrane. Tail represents the detection protein loaded on the intracellular side of the membrane. In effect, each module consists of nucleic acid molecules encoding the respective proteins/functional domains.

Preferably, the effector module comprises a nucleotide sequence/nucleic acid molecule encoding an effector protein or a functional protein.

Preferably, the effector module comprises any one or more selected from the group consisting of single chain interleukin 12 (scIL12), CD47, apolipoprotein E (APOE), CD24, ScFv, GDNF, and CD40L.

Preferably, the scaffold module comprises any one or more selected from the group consisting of Fc, Foldon, Fc+Ig1 to Ig3 domains of NPTN (NPTN-Ig1-3), I-EGF1 to I-EGF4 domains of ITGB1 (ITGB1-I-EGF1-4), Foldon+NPTN-Ig1-3, and NPTN-Ig1-3.

Preferably, the transmembrane domain module comprises any one or more selected from the group consisting of TMD of EWI-F, TMD of EWI-2, TMD of ITGB1, TMD_Mut_10, TMD_Mut_13, TMD_Mut_17, and NPTN-TMD.

Preferably, the transmembrane domain module comprises any one or more selected from the group consisting of TMD_Mut_10, TMD_Mut_13, TMD_Mut_17, and NPTN-TMD;
wherein TMD_Mut_10 has a nucleotide sequence that encodes an amino acid sequence set forth in SEQ ID NO. 5,
TMD_Mut_13 has a nucleotide sequence that encodes an amino acid sequence set forth in SEQ ID NO. 8,
TMD_Mut_17 has a nucleotide sequence that encodes an amino acid sequence set forth in SEQ ID NO. 9, and
NPTN-TMD has a nucleotide sequence that encodes an amino acid sequence set forth in SEQ ID NO. 10.

Preferably, the intracellular domain anchoring module comprises a nucleotide sequence encoding S-palmitoyl cysteine, and the intracellular domain anchoring module comprises the intracellular domain (ICD) of EWI-F (EWF-F-ICD) or the ICD of EWI-2 (EWI-2-ICD).

Preferably, the detection module comprises any one or more of a fluorescent protein module and/or a chemiluminescent module.

Preferably, the fluorescent protein module comprises any one or more selected from the group consisting of green fluorescent protein, red fluorescent protein, blue fluorescent protein, and yellow fluorescent protein; and the chemiluminescent module comprises nanoLuciferase (nanoLuc) or Rluciferase (RLuc).

In a second aspect, the present disclosure provides a transmembrane domain mutant protein, and the transmembrane domain mutant protein comprises any one selected from the group consisting of TMD_Mut_10, TMD_Mut_13, and TMD_Mut_17;
wherein TMD_Mut_10 has a nucleotide sequence that encodes an amino acid sequence set forth in SEQ ID NO. 5,
TMD_Mut_13 has an amino acid sequence set forth in SEQ ID NO. 8, and
TMD_Mut_17 has an amino acid sequence set forth in SEQ ID NO. 9.

In a third aspect, the present disclosure provides a nucleic acid construct comprising a nucleic acid molecule encoding the transmembrane domain mutant protein according to the second aspect.

Preferably, the nucleic acid construct further comprises an effector module (Head), a scaffold module (Scaffold), a transmembrane domain module (TMD), and an intracellular domain anchoring module (ICD);
wherein the effector module, the scaffold module, the transmembrane domain module and the intracellular domain anchoring module are sequentially connected from 5' end to 3' end either directly or through a linking module, and the linking module comprises a linker peptide (Linker).

The linker peptide is classified into rigid and flexible linker peptides. Common sequences of rigid linker peptides include (EAAAK)ₙ (n=1-3) and common sequences of flexible linker peptides include (GGGGS)ₙ (n=1-5).

Preferably, a flexible linker peptide is used between the modules of the present disclosure.

Preferably, the liner peptide of the present disclosure has an amino acid sequence of (GGGGS)₃.

Preferably, the nucleic acid construct further comprises a detection module (Tail), wherein the 5' end of the detection module is connected to the 3' end of the intracellular domain anchoring module.

Preferably, the effector module comprises any one or more selected from the group consisting of single chain interleukin 12 (scIL12), CD47, apolipoprotein E (APOE), hCD24, ScFv, GDNF, and CD40L.

Preferably, the scaffold module comprises any one or more selected from the group consisting of Fc, Foldon, Fc+NPTN-Ig1-3, ITGB1-I-EGF1-4, Foldon+NPTN-Ig1-3, and NPTN-Ig1-3.

Preferably, the intracellular domain anchoring module comprises a nucleotide sequence encoding S-palmitoyl cysteine, and the intracellular domain anchoring module comprises EWI-F-ICD or EWI-2-ICD.

Preferably, the detection module comprises any one or more of a fluorescent protein module and/or a chemiluminescent module.

Preferably, the fluorescent protein module comprises any one or more selected from the group consisting of green fluorescent protein, red fluorescent protein, blue fluorescent protein, and yellow fluorescent protein; and the chemiluminescent module comprises nanoLuciferase (nanoLuc) or Rluciferase (RLuc).

In a fourth aspect, the present disclosure provides a plasmid, expression vector, or expression cassette comprising the nucleic acid construct according to the first aspect or third aspect.

In a fifth aspect, the present disclosure provides a transformant or recombinant cell comprising the plasmid, expression vector or expression cassette according to the fourth aspect.

In a sixth aspect, the present disclosure provides an engineered EV prepared using the transformant or recombinant cell according to the fifth aspect.

In a seventh aspect, the present disclosure provides a protein expressed by the nucleic acid construct according to the first aspect or third aspect.

In an eighth aspect, the present disclosure provides use of the nucleic acid construct according to the first aspect or third aspect, the plasmid, expression vector or expression cassette according to the fourth aspect, the transformant or recombinant cell according to the fifth aspect, the engineered EV according to the sixth aspect, or the protein according to the seventh aspect in the manufacture of a medicament or an agent, wherein the medicament has the effector module as an active ingredient.

In the present disclosure, the effector module comprises any one or more selected from the group consisting of scIL12, CD47, APOE, hCD24, ScFv, GDNF and CD40L.

The scIL12 refers to Interleukin-12, a pro-inflammatory cytokine in a heterodimeric form produced by antigen-presenting cells and B cells and secreted in this heterodimeric form. IL12 primarily acts on T cells and NK cells, inducing the production of IFN-γ and regulating immune processes. It is used in tumor immunotherapy.

CD47, also known as Integrin-Associated Protein (IAP), is a typical "marker of self" expressed on all cells. CD47 is a key regulator of tissue homeostasis and plays a role in various diseases ranging from atherosclerosis to cancer. CD47 protects host cells by evading phagocytosis. CD47 expressed by cancer cells interacts with SIRPα and SIRPγ of SIRP protein family expressed by NK cells. CD47 and its ligands are involved in cell adhesion, cell migration, phagocytosis, and maintaining immune homeostasis.

APOE, or Apolipoprotein E, is a polymorphic protein involved in lipoprotein conversion and metabolism. Its gene regulates various biological functions and is associated with several ophthalmic diseases.

hCD24, or human Cluster of Differentiation 24, also known as Heat-Stable Antigen, is a heavily glycosylated glycosylphosphatidylinositol-anchored surface protein. It interacts with Siglec-10 (sialic-acid-binding Ig-like lectin 10) on innate immune cells to suppress destructive inflammatory responses to infections, sepsis, liver damage, and chronic graft-versus-host disease.

ScFv, short for single-chain variable fragment, is composed of the variable regions of the antibody heavy and light chains linked by a short peptide of 15-20 amino acids.

GDNF, or Glial Cell Line-Derived Neurotrophic Factor, is found to be expressed in cultures of various neuronal and neuro-related cells. It acts as a neurotrophic factor with target-specific properties.

CD40L, also known as gp39, TNF-Associated Activation Protein (TRAP), or T-BAM (T Cell-B Cell Activating Molecule), is a type II transmembrane glycoprotein. It is expressed on activated and infiltrating T cells, as well as on B cells, platelets, dendritic cells (DCs), monocytes under inflammatory conditions, natural killer cells, mast cells, and eosinophils. It is also expressed in tumor cells.

In the present disclosure, the scaffold module comprises any one or more selected from the group consisting of Fc, Foldon, Fc+NPTN-Ig1-3, ITGB1-I-EGF1-4, Foldon+NPTN-Ig1-3, and NPTN-Ig1-3.

Fc refers to a segment of the antibody heavy chain constant region, containing the common protein sequence of all antibody molecules and unique determinants for each class. It has various biological activities, such as complement binding, Fc receptor binding, and placental transfer. Fc fragments can form dimers through disulfide bonds and are often used as specific tags in chromatography.

Foldon is a small β-propeller-like trimer of 27 residues, composed of β-hairpin units. It was initially identified at the C-terminus of the bacteriophage T4 fiber protein. When fused with a target protein, Foldon spontaneously forms a stable trimeric structure.

ITGB1, or Integrin Subunit Beta 1, is an integrin β1 subunit. Integrins are widely expressed adhesion molecules, and as cell surface receptors, exist as α and β subunit heterodimers. They are involved in cell adhesion and recognition during embryogenesis, hemostasis, tissue repair, immune response, and tumor cell metastasis. The β1 subunit is a type I single-pass transmembrane protein involved in the adhesion of exosomes.

ITGB1-I-EGF1-4 refers to the four I-EGF repeat sequences on the extracellular side of the membrane of the integrin β1 subunit.

ITGB1-TMD refers to the transmembrane sequence of the integrin β1 subunit transmembrane protein.

NPTN, or Neuroplastin, is a cell adhesion molecule and a type I transmembrane protein of the immunoglobulin superfamily. NPTN, along with BSG and Embigin (gp70), forms the immunoglobulin superfamily, also known as CD147 family. This family has extracellular domains, transmembrane domains, and intracellular domains in its structure. NPTN and BSG share 40-50% structural homology, with the highest homology in the transmembrane domain (including the charged glutamate residue) and intracellular domain.

NPTN-Ig1-3 refers to three Ig-like extracellular sequences of the NPTN transmembrane protein.

In the present disclosure, the intracellular domain anchoring module comprises a nucleotide sequence encoding S-palmitoyl cysteine, and the intracellular domain anchoring module comprises EWI-F-ICD or EWI-2-ICD

EWI-F refers to Prostaglandin F2 Receptor Negative Regulator, which inhibits the binding of Prostaglandin F2α (PGF2α) to its specific receptor by reducing receptor numbers. In myoblasts, EWI-F binds with CD9 and CD81 to prevent myotube fusion during muscle regeneration. EWI-F is a single-pass transmembrane protein present on the exosome membrane surface, binding with CD9.

EWI-2 refers to Immunoglobulin Superfamily Member 8, which regulates cell proliferation and differentiation. It plays a crucial role in various functions of CD9 and CD81, suppressing T cell migration in coordination with CD81 and inhibiting prostate cancer cell migration with CD82. EWI-2 is a single-pass transmembrane protein present on the exosome membrane surface, binding with CD81.

In the present disclosure, the chemiluminescent module comprises Nanoluc or Rluc.

Nanoluc is a novel reporter gene of luciferase, NanoLuc luciferase, modified from shrimp, designed through directed evolution and new substrate development. It is a small (19 kDa) monomeric enzyme with unique substrates, offering about 100 times greater sensitivity than the highly sensitive firefly or Renilla luciferase systems. This new reporter gene has broad application prospects, laying the foundation for further technical development.

RLuc, or Renilla luciferase, is a luciferase from the marine cnidarian Renilla reniformis. It is a 36 kD monomeric polypeptide chain with 311 amino acid residues. It is a widely used marine luciferase, catalyzing the production of chemiluminescence from coelenterazine with an emission wavelength around 480 nm. Although its quantum yield is lower (7%) compared to FLuc, it does not require ATP or other cofactors. As a result, it has fewer reaction limitations and is widely used in bioluminescence detection techniques based on luciferase.

The present disclosure adopts a modular design to construct a nucleic acid construct for modular loading of a functional protein into engineered EVs. Peptide sequences for purification (Tag) can be introduced at the 5' end of the nucleic acid construct to achieve the specific enrichment of EVs. Additionally, scaffold proteins such as Fc or Foldon, which can form dimers or trimers, can be incorporated to ultimately achieve modular loading of functional proteins and enhance the loading efficiency. Scaffold sequences like ITGB1 and NPTN are used to prevent enzyme cleavage (such as the cleavage of EWI-F by ADAM10). Proteins are loaded onto the EV membrane via the transmembrane domain and intracellular domain anchoring modules. By knocking out the background transmembrane protein genes, the loading density of each modular protein on the EVs can be increased.

The amino acid sequence of CD9 is set forth in SEQ ID NO. 1:

MPVKGGTKCIKYLLFGFNFIFWLAGIAVLAIGLWLRFDSQTKSIFEQETNNNNNSSFYTG

VYILIGAGALMMLVGFLGCCGAVQESQCMLGLFFGFLLVIFAIEIAAAIWGYSHKDEV
IKEVQEFYKDTYNKLKTKDEPQRETLKAIHYALNCCGLAGGVEQFISDICPKKDVLETFT
VKSCPDAIKEVFDNKFHIIGAVGIGIAVVMIFGMIFSMILCCAIRRNREMV

CD9 has four transmembrane domains, as indicated by the bold and underlined portions of the sequence. The specific sequences are:
LLFGFNFIFWLAGIAVLAIGL
FYTGVYILIGAGALMMLVGFL
MLGLFFGFLLVIFAIEIAAAIWGY
MLGLFFGFLLVIFAIEIAAAIWGY

The amino acid sequence of EWI-F-TMD is set forth in SEQ ID NO. 2: LLIGVGLSTVIGLLSCLIGYCSS

The amino acid sequence of EWI-2-TMD is set forth in SEQ ID NO. 3: LFVPLLVGTGVALVTGATVLG

The amino acid sequence of BSG-TMD is set forth in SEQ ID NO. 4: ALWPFLGIVAEVLVLVTIIFI

The amino acid sequence of scIL12 is set forth in SEQ ID NO. 6:

IWELKKDVYVVELDWYPDAPGEMVVLTCDTPEEDGITWTLDQSSEVLGSGKTL
TIQVKEFGDAGQYTCHKGGEVLSHSLLLLHKKEDGIWSTDILKDQKEPKNKTFLRCEAK
NYSGRFTCWWLTTISTDLTFSVKSSRGSSDPQGVTCGAATLSAERVRGDNKEYEYSVECQ
EDSACPAAEESLPIEVMVDAVHKLKYENYTSSFFIRDIIKPDPPKNLQLKPLKNSRQVEVS
WEYPDTWSTPHSYFSLTFCVQVQGKSKREKKDRVFTDKTSATVICRKNASISVRAQDRY
YSSSWSEWASVPCSGGGGSGGGGSGGGGSRNLPVATPDPGMFPCLHHSQNLLRAVSNML
QKARQTLEFYPCTSEEIDHEDITKDKTSTVEACLPLELTKNESCLNSRETSFITNGSCLASR
KTSFMMALCLSSIYEDLKMYQVEFKTMNAKLLMDPKRQIFLDQNMLAVIDELMQALNF
NSETVPQKSSLEEPDFYKTKIKLCILLHAFRIRAVTIDRVMSYLNAS

The amino acid sequence of Nanoluc is set forth in SEQ ID NO. 7:

VFTLEDFVGDWRQTAGYNLDQVLEQGGVSSLFQNLGVSVTPIQRIVLSGENGL

KIDIHVIIPYEGLSGDQMGQIEKIFKVVYPVDDHHFKVILHYGTLVIDGVTPNMIDYFGRP
YEGIAVFDGKKITVTGTLWNGNKIIDERLINPDGSLLFRVTINGVTGWRLCER

The amino acid sequence of TMD_Mut_10 is set forth in SEQ ID NO. 5:
LLIGVNLLTVIGLWLFLILYCSL

The amino acid sequence of TMD_Mut_13 is set forth in SEQ ID NO. 8:
LLIGVGLLTVIGLLLFLILYCSS

The amino acid sequence of TMD_Mut_17 is set forth in SEQ ID NO. 9:
LLIGVGLSTVIGLLSCLISYCSS

The amino acid sequence of NPTN-TMD is set forth in SEQ ID NO. 10:
LWPFLGILAEIIILVVIIVVY

The amino acid sequence of EGFP is set forth in SEQ ID NO. 11:

MVSKGEELFTGVVPILVELDGDVNGHKFSVSGEGEGDATYGKLTLKFICTTGKL
PVPWPTLVTTLTYGVQCFSRYPDHMKQHDFFKSAMPEGYVQERTIFFKDDGNYKTRAEV
KFEGDTLVNRIELKGIDFKEDGNILGHKLEYNYNSHNVYIMADKQKNGIKVNFKIRHNIE
DGSVQLADHYQQNTPIGDGPVLLPDNHYLSTQSALSKDPNEKRDHMVLLEFVTAAGITL
GMDELYK

The amino acid sequence of Fc is set forth in SEQ ID NO. 12:

AESKYGPPCPPCPAPEAAGGPSVFLFPPKPKDTLMISRTPEVTCVVVDVSQEDPE
VQFNWYVDGVEVHNAKTKPREEQFNSTYRVVSVLTVLHQDWLNGKEYKCKVSNKGLP
SSIEKTISKAKGQPREPQVYTLPPSQEEMTKNQVSLTCLVKGFYPSDIAVEWESNGQPENN
YKTTPPVLDSDGSFFLYSRLTVDKSRWQEGNVFSCSVMHEALHNHYTQKSLSLSLG

The amino acid sequence of hCD24 is set forth in SEQ ID NO. 13:

MGRAMVARLGLGLLLLALLLPTQIYSSETTTGTSSNSSQSTSNSGLAPNPTNATT
KAAG

The amino acid sequence of Rluc is set forth in SEQ ID NO. 14:

ASKVYDPEQRKRMITGPQWWARCKQMNVLDSFINYYDSEKHAENAVIFLHGN

AASSYLWRHVVPHIEPVARCIIPDLIGMGKSGKSGNGSYRLLDHYKYLTAWFELLNLPKKI
IFVGHDWGACLAFHYSYEHQDKIKAIVHAESVVDVIESWDEWPDIEEDIALIKSEEGEKM
VLENNFFVETMLPSKIMRKLEPEEFAAYLEPFKEKGEVRRPTLSWPREIPLVKGGKPDVV
QIVRNYNAYLRASDDLPKMFIESDPGFFSNAIVEGAKKFPNTEFVKVKGLHFSQEDAPDE
MGKYIKSFVERVLKNEQ

The amino acid sequence of Fc+NPTN-Ig1-3 is set forth in SEQ ID NO. 15:

AESKYGPPCPPCPAPEAAGGPSVFLFPPKPKDTLMISRTPEVTCVVVDVSQEDPE
VQFNWYVDGVEVHNAKTKPREEQFNSTYRVVSVLTVLHQDWLNGKEYKCKVSNKGLP
SSIEKTISKAKGQPREPQVYTLPPSQEEMTKNQVSLTCLVKGFYPSDIAVEWESNGQPEN
NYKTTPPVLDSDGSFFLYSRLTVDKSRWQEGNVFSCSVMHEALHNHYTQKSLSLSLGQN
AGFVKSPMSETKLTGDAFELYCDVVGSPTPEIQWWYAEVNRAESFRQLWDGARKRRVT
VNTAYGSNGVSVLRITRLTLEDSGTYECRASNDPKRNDLRQNPSITPRIVTSEEVIIRDSPV
LPVTLQCNLTSSSHTLTYSYWTKNGVELSATRKNASNMEYRINKPRAEDSGEYHCVYHF
VSAPKANATIEVKPDITGHKRSENKNEGQDATMYCKSVGYPHPDWIWRKKENGMPMDI
VNTSGRFFIINKENYTELNIVNLQITEDPGEYECNATNAIGSASVVT

The amino acid sequence of ITGB1-I-EGF1-4 is set forth in SEQ ID NO. 16:

CQSEGIPESPKCHEGNGTFECGACRCNEGRVGRHCECSTDEVNSEDMDAYCRK
ENSSEICSNNGECVCGQCVCRKRDNTNEIYSGKFCECDNFNCDRSNGLICGGNGVCKCR
VCECNPNYTGSACDCSLDTSTCEASNGQICNGRGICECGVCKCTDPKFQGQTCEMCQT

The amino acid sequence of Foldon+NPTN-Ig1-3 is set forth in SEQ ID NO. 17:

YIPEAPRDGQAYVRKDGEWVLLSTFLGQNAGFVKSPMSETKLTGDAFELYCDV
VGSPTPEIQWWYAEVNRAESFRQLWDGARKRRVTVNTAYGSNGVSVLRITRLTLEDSGT
YECRASNDPKRNDLRQNPSITPRIVTSEEVIIRDSPVLPVTLQCNLTSSSHTLTYSYWTKNG
VELSATRKNASNMEYRINKPRAEDSGEYHCVYHFVSAPKANATIEVKPDITGHKRSENK
NEGQDATMYCKSVGYPHPDWIWRKKENGMPMDIVNTSGRFFIINKENYTELNIVNLQIT
EDPGEYECNATNAIGSASVVT

The amino acid sequence of CD47 is set forth in SEQ ID NO. 18:

QLLFNKTKSVEFTFCNDTVVIPCFVTNMEAQNTTEVYVKWKFKGRDIYTFDGA

LNKSTVPTDFSSAKIEVSQLLKGDASLKMDKSDAVSHTGNYTCEVTELTREGETIIELKYR
VVSW

The amino acid sequence of CD40L is set forth in SEQ ID NO. 19:

GDQNPQIAAHVISEASSKTTSVLQWAEKGYYTMSNNLVTLENGKQLTVKRQGL
YYIYAQVTFCSNREASSQAPFIASLCLKSPGRFERILLRAANTHSSAKPCGQQSIHLGGVFE
LQPGASVFVNVTDPSQVSHGTGFTSFGLLKL

The amino acid sequence of APOE is set forth in SEQ ID NO. 20:

KVEQAVETEPEPELRQQTEWQSGQRWELALGRFWDYLRWVQTLSEQVQEELL
SSQVTQELRALMDETMKELKAYKSELEEQLTPVAEETRARLSKELQAAQARLGADMED
VCGRLVQYRGEVQAMLGQSTEELRVRLASHLRKLRKRLLRDADDLQKRLAVYQAGARE
GAERGLSAIRERLGPLVEQGRVRAATVGSLAGQPLQERAQAWGERLRARMEEMGSRTR
DRLDEVKEQVAEVRAKLEEQAQQIRLQAEAFQARLKSWFEPLVEDMQRQWAGLVEKVQ
AAVGTSAAPVPSDNH

The amino acid sequence of Anti-CD3-ScFv(L-H) is set forth in SEQ ID NO. 21:

QTVVTQEPSLTVSPGGTVTLTCGSSTGAVTSGNYPNWVQQKPGQAPRGLIGGTK
FLAPGTPARFSGSLLGGKAALTLSGVQPEDEAEYYCVLWYSNRWVFGGGTKLTVLGGGG
SGGGGSGGGGSEVQLVESGGGLVQPGGSLKLSCAASGFTFNKYAMNWVRQAPGKGLE
WVARIRSKYNNYATYYADSVKDRFTISRDDSKNTAYLQMNNLKTEDTAVYYCVRHGNFG
NSYISYWAYWGQGTLVTVSS

The amino acid sequence of Anti-CLDN18.2-ScFv(L-H) is set forth in SEQ ID NO. 22:

DIQMTQSPSSLSASVGDRVTITCKSSQSLFNTGNQKNYLTWYQQKPGKAPKLLI
YWASTRESGVPSRFSGSGSGTDFTLTISSLQPEDFATYYCQNNYNFPLTFGQGTRLEIKGG
GGSGGGGSGGGGSQVQLVESGGGLVKPGGSLRLSCAASGFSFSSFGMHWVRQAPGKGL
EWVAYISSGSRTIYYADTVKGRFTISRDNAKNSLYLQMNSLRAEDTAVYYCTRYYYGNSF
DYWGQ GTTVTVSS

The amino acid sequence of Anti-GPC3-ScFv(L-H) is set forth in SEQ ID NO. 23:

DVVMTQSPLSLPVTPGEPASISCRSSQSLVHSNANTYLHWYLQKPGQSPQLLIYK
VSNRFSGVPDRFSGSGSGTDFTLKISRVEAEDVGVYYCSQNTHVPPTFGQGTKLEIKGGG

GSGGGGSGGGGSQVQLVQSGAEVKKPGASVKVSCKASGYTFTDYEMHWVRQAPGQGL
EWMGALDPKTGDTAYSQKFKGRVTLTADESTSTAYMELSSLRSEDTAVYYCTRFYSYTY
WGQGTLVTVSS

The amino acid sequence of GDNF is set forth in SEQ ID NO. 24:

MKLWDVVAVCLVLLHTASAFPLPAGKRPPEAPAEDRSLGRRRAPFALSSDSNMP
EDYPDQFDDVMDFIQATIKRLKRSPDKQMAVLPRRERNRQAAAANPENSRGKGRRGQR
GKNRGCVLTAIHLNVTDLGLGYETKEELIFRYCSGSCDAAETTYDKILKNLSRNRRLVSD
KVGQACCRPIAFDDDLSFLDDNLVYHILRKHSAKRCGCI.

The amino acid sequence of EWI-F-ICD is set forth in SEQ ID NO.25:
HWCCKKEVQETRRERRRLMSMEMD

The amino acid sequence of EWI-2-ICD is set forth in SEQ ID NO.26:
TITCCFMKRLRKR

The amino acid sequence of ITGB1-TMD is set forth in SEQ ID NO.27:
IIPIVAGVVAGIVLIGLALLLIW

As used herein, the term "extracellular vesicles" or "EVs" refers to cell-derived vesicles that comprise a membrane enclosing an internal space (i.e., lumen). Extracellular vesicles include all membrane-bound vesicles with a diameter smaller than that of their originating cell. Typically, extracellular vesicles vary in diameter from 20 nm to 1000 nm, and can comprise various payloads within the internal space (i.e., lumen), displayed on the external surface or on the lumen surface of the extracellular vesicle, and/or across the membrane. Payloads may include, for example, nucleic acids, proteins, carbohydrates, lipids, small molecules, and/or combinations thereof. The extracellular vesicles may be microvesicles or exosomes. The extracellular vesicles may originate from any suitable cell and include, but are not limited to, apoptotic bodies, cellular debris, vesicles derived from cells through direct or indirect manipulation (e.g., through continuous extrusion or treatment with alkaline solutions), vesiculated organelles, and vesicles produced by living cells (e.g., by direct budding from the plasma membrane or fusion of late endosomes with the plasma membrane). Extracellular vesicles can be derived from living or dead organisms, explanted tissues or organs, and/or cultured cells.

As described herein, engineering can be applied directly to the lumen (i.e., the void within the EVs) or to the membrane of the EVs (e.g., exosomes), particularly to the luminal surfaces of the EVs, thereby altering the lumen and/or luminal surface of EVs (e.g., exosomes). For example, the composition of proteins, lipids, small molecules, carbohydrates, etc., in the membrane can be modified, thereby altering the inner luminal surface of the vesicles. Similarly, the contents of the lumen can be modified. Composition can be altered through chemical, physical, or biological methods, or by using cells that have been previously modified by chemical, physical, or biological methods. Specifically, composition can be altered through genetic engineering or by using cells that have been genetically engineered. In some aspects, lumen-engineered EVs (e.g., lumen-engineered exosomes) contain exogenous proteins (i.e., proteins not naturally expressed by EVs (e.g., exosomes)) or their fragments or variants, which can be exposed on the inner surface or within the lumen of EVs (e.g., exosomes), or can serve as anchoring points (attachment) exposed on parts of the inner surface of EVs (e.g., exosomes). In some other aspects, lumen-engineered EVs (e.g., lumen-engineered exosomes) have higher expression of native EV (e.g., exosome) proteins (e.g., scaffold proteins) or their fragments or variants, which can be exposed within the lumen of EVs (e.g., exosomes), or can serve as anchoring points (attachment) exposed on parts of the inner surface of EVs (e.g., exosomes).

### Beneficial Effects:

The nucleic acid construct for modular loading of a functional protein into engineered EVs and use thereof provided by the present disclosure offer the following beneficial effects:

By employing a modular design principle, the present disclosure selects and combines specific modules and optimizes screened mutation sequences to construct an improved nucleic acid construct for modular loading of a functional protein into engineered EVs, and finally obtains engineered EVs modularly loaded with functional protein. The product of the present disclosure has the following advantages: 1) achievement of modular loading of functional proteins in engineered EVs; 2) enhanced loading density and efficiency of proteins on and within the EV membranes by utilizing specific transmembrane domain sequences that synergistically interact with other modules; and 3) protection from enzyme cleavage by utilizing specific scaffold sequence; and 4) versatility in regard to oligomerization state of the functional proteins to be loaded. The nucleic acid construct obtained in the present disclosure enable the preparation of engineered EVs capable of modular loading of functional proteins, providing significant clinical application value and market prospect.

### BRIEF DESCRIPTION OF DRAWINGS

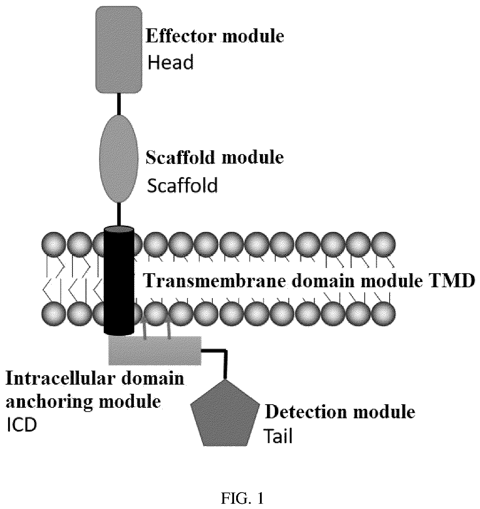

FIG. 1 shows the structural schematic diagram of the nucleic acid construct of the present disclosure.
FIG. 2 shows the structural model of the CD9-EWI-F-TMD complex of the present disclosure; the grid model represents the density map obtained from cryo-electron microscopy.
FIG. 3 shows the structural model of the CD9-EWI-2-TMD complex of the present disclosure; the grid model represents the density map obtained from cryo-electron microscopy.
FIG. 4 shows the structural model of the CD9-BSG-TMD complex of the present disclosure; the grid model represents the density map obtained from cryo-electron microscopy.
FIG. 5 shows the structural model showing the strongest binding between CD9 and TMD in the present disclosure.
FIG.6 shows the EGFP loading results in the present disclosure, where the left panel shows the detection results of EGFP positivity rate at the cellular level, and the right panel shows the detection results of EGFP positivity rate at the EV level.
FIG. 7 shows the Nanoluc enzyme activity at the cellular level in the present disclosure.
FIG. 8 shows the standard curve of enzyme activity of Nanoluc loaded by EVs in the present disclosure.
FIG. 9 shows the Rluc enzyme activity at the cellular level in the present disclosure.
FIG. 10 shows the standard curve of enzyme activity of Rluc loaded by EVs in the present disclosure.
FIG. 11 shows the Western Blot detection of Fc loading in the present disclosure.
FIG. 12 shows the chromatogram of Fc-tagged EVs enriched using protein A in the present disclosure.
FIG. 13 shows the Western Blot detection of NPTN-Ig1-3 loading in the present disclosure.
FIG. 14 shows the Western Blot detection of ITGB1-I-EGF1-4 loading in the present disclosure.
FIG. 15 shows the Western Blot detection of CD40L loading in the present disclosure.
FIG. 16 shows the binding curve of APOE-EVs to LRP proteins detected by Biacore-T200 in the present disclosure.
FIG. 17 shows the Western Blot detection of hCD24 loading in the present disclosure.
FIG. 18 shows the ELISA detection of the efficacy of hCD24-Evs on PBMCs after loading in the present disclosure.
FIG. 19 shows the NanoFCM and Western Blot detection of Anti-CD3-ScFv(L-H) loading in the present disclosure.
FIG. 20 shows the ELISA detection of the efficacy of Anti-CD3-ScFv(L-H)-EVs on PBMCs after loading in the present disclosure.
FIG. 21 shows the Western Blot detection of Anti-CLDN18.2-ScFv(L-H) loading in the present disclosure.
FIG. 22 shows the Nanoluc enzyme activity detection of Anti-CLDN18.2-ScFv(L-H)-EVs after loading in the present disclosure.
FIG. 23 shows the Western Blot detection of Anti-GPC3-ScFv(L-H) loading in the present disclosure.
FIG. 24 shows binding curves of Anti-GPC3-ScFv(L-H) EVs of the present disclosure to antigen.
FIG. 25 shows the Nanoluc enzyme activity detection of GDNF-EVs after loading in the present disclosure.
FIG. 26 shows the flow cytometry detection of CD40L expression at the cellular level in the present disclosure.
FIG. 27 shows the Nanoluc enzyme activity detection of CD40L-EVs after loading in the present disclosure.
FIG. 28 shows the effect of CD40L-EVs on B cell proliferation after loading in the present disclosure.
FIG. 29 shows the effect of CD40L-EVs on B cell activation marker expression after loading in the present disclosure.
FIG. 30 shows the NanoFCM detection of GFP positivity rate in CD47-EVs after loading in the present disclosure.
FIG. 31 shows the Nanoluc enzyme activity detection of CD47-EVs after loading in the present disclosure.
FIG. 32 shows the Nanoluc enzyme activity detection of scIL2-EVs after loading in the present disclosure.
FIG. 33 shows the ELISA detection of the efficacy of scIL2-EVs on PBMCs after loading in the present disclosure.

### DETAILED DESCRIPTION

The present disclosure disclosed a nucleic acid construct for achieving modular loading of a functional protein into engineered EVs and use thereof. Those skilled in the art can learn from the contents of the present disclosure and appropriately improve the processing parameters. In particular, it should be noted that all similar replacements and modifications are apparent to those skilled in the art, which are all considered to be included in the present disclosure. The method and the application of the present disclosure have been described through the preferred embodiments, and it is obvious that the method and application described herein may be changed or appropriately modified and combined to realize and apply the technology of the present disclosure by those skilled in the art without departing from the content, spirit and scope of the present disclosure.

The materials and reagents used in the nucleic acid construct for achieving modular loading of a functional protein into engineered EVs and use thereof provided by the present disclosure are commercially available.

The present disclosure is further illustrated below in conjunction with examples.

### Example 1: Design and Optimization of Transmembrane Domain (TMD) Sequences

### 1. Construction of the 3D Structure Models of the CD9-EWI-F/EWI-2/BSG Transmembrane Domain Complex

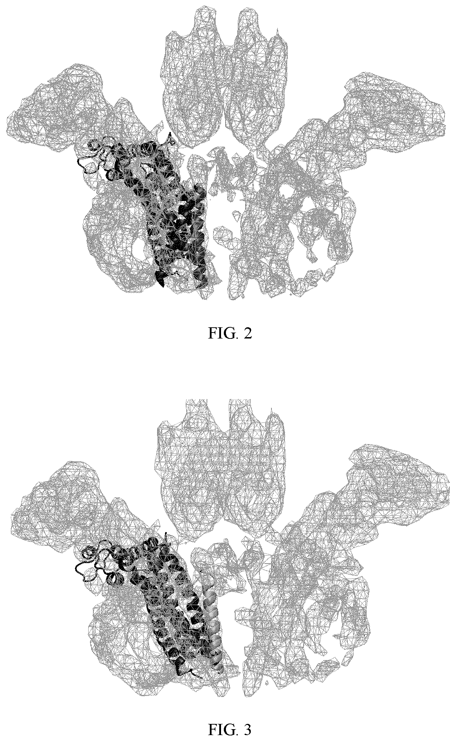

Based on the published cryo-electron microscopy density data for the CD9-EWI-F protein complex (www.ebi.ac.uk/emdb/EMD-11053), the 3D structure model of CD9 (PDB ID: 6K4J) and the transmembrane domain (TMD) models of the proteins EWI-F, EWI-2, and BSG were inserted into the cryo-EM density map. The modeling results are shown in Figures 2 to 4.

The amino acid sequence of CD9 is set forth in SEQ ID NO. 1:

MPVKGGTKCIKYLLFGFNFIFWLAGIAVLAIGLWLRFDSQTKSIFEQETNNNNNSSFYTG
VYILIGAGALMMLVGFLGCCGAVQESQCMLGLFFGFLLVIFAIEIAAAIWGYSHKDEV
IKEVQEFYKDTYNKLKTKDEPQRETLKAIHYALNCCGLAGGVEQFISDICPKKDVLETFT
VKSCPDAIKEVFDNKFHIIGAVGIGIAVVMIFGMIFSMILCCAIRRNREMV

The amino acid sequence of EWI-F-TMD is set forth in SEQ ID NO. 2: LLIGVGLSTVIGLLSCLIGYCSS

The amino acid sequence of EWI-2-TMD is set forth in SEQ ID NO. 3: LFVPLLVGTGVALVTGATVLG

The amino acid sequence of BSG-TMD is set forth in SEQ ID NO. 4: ALWPFLGIVAEVLVLVTIIFI

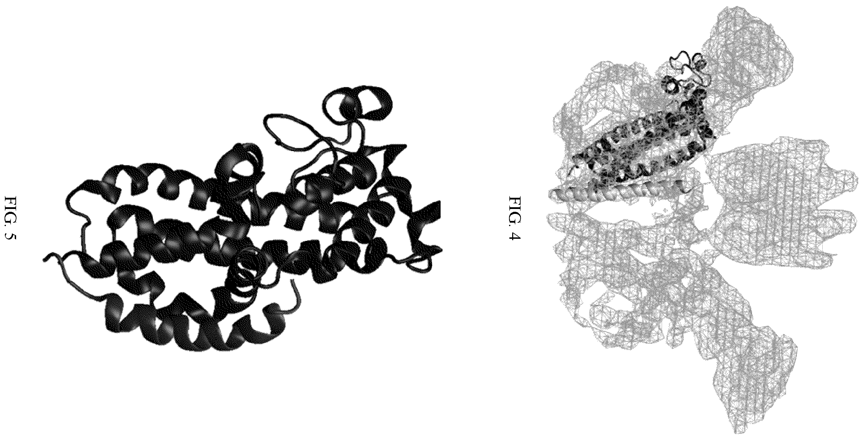

The structural models of these three complexes were subjected to 10 ns molecular dynamics simulations under vacuum conditions (simulating a low dielectric environment of the cell membrane) using NAMD molecular dynamics simulation software. The interaction energy between CD9 and TMD in each frame was calculated using namd_energy software, and the structure model with the strongest interaction (lowest energy) (CD9-EWI-F-TMD) was selected, as shown in FIG. 5.

### 2. Artificial Intelligence-Based Optimization of TMD Sequences

Mutagenesis designs for the EWI-F/EWI-2-TMD sequence in the CD9-EWI-F/EWI-2-TMD complex structure were performed using artificial intelligence protein sequence prediction algorithms combined with AutoDock Vina software. The amino acid sequence of the wild-type, unmutated EWI-F-TMD is set forth in SEQ ID NO. 2:
LLIGVGLSTVIGLLSCLIGYCSS

The amino acid sequence of the wild-type, unmutated EWI-2-TMD is set forth in SEQ ID NO. 3:
LFVPLLVGTGVALVTGATVLG

A total of 20 TMD sequences with strong potential binding activity to CD9 were identified (where numbers 1-17 are mutated sequences of EWI-F-TMD, and numbers 18-20 are mutated sequences of EWI-2-TMD), as shown in Table 1.

**Table 1. 20 Different Mutant TMDs**

| No. | Mutation sites |
|---|---|
| TMD_Mut_1 | G6K, T9R, S22L, S23N |
| TMD_Mut_2 | G6K, T9R, S23N |
| TMD_Mut_3 | S22L |
| TMD_Mut_4 | G6K, T9R, S22L |
| TMD_Mut_5 | S22L, S23N |
| TMD_Mut_6 | G6R, S8I, S15H, C16L |
| TMD_Mut_7 | S8I, C16L |
| TMD_Mut_8 | G6R, S15H |
| TMD_Mut_9 | G6R, S8I, S15H |
| TMD_Mut_10 | G6N, S8L, L14W, S15L, C16F, G19L, S23L |
| TMD_Mut_11 | G6N, L14W, C16F |
| TMD_Mut_12 | S8L, S15L, G19L, S23L |
| TMD_Mut_13 | S8L, S15L, C16F, G19L |
| TMD_Mut_14 | G6N, L14W, C16F, S23L |
| TMD_Mut_15 | G6Q, S15R, G19S |
| TMD_Mut_16 | G6Q, S15R |
| TMD_Mut_17 | G19S |
| TMD_Mut_18 | L1Y, F2K |
| TMD_Mut_19 | A12S |
| TMD_Mut_20 | L1Y, F2K, A12S |

### 3. Verification of EV Loading with Optimized TMD Sequences

### Steps:

1) Plasmids were constructed using pscIL12-TMD(EWI-F/EWI-2-TMD)-ICD(EWI-F-ICD)-Nanoluc as the vector, and the transmembrane domain sequences were replaced with the above 20 mutant transmembrane domain sequences and the NPTN-TMD sequence.

The amino acid sequence of scIL12 is set forth in SEQ ID NO. 6:

IWELKKDVYVVELDWYPDAPGEMVVLTCDTPEEDGITWTLDQSSEVLGSGKTL
TIQVKEFGDAGQYTCHKGGEVLSHSLLLLHKKEDGIWSTDILKDQKEPKNKTFLRCEAK
NYSGRFTCWWLTTISTDLTFSVKSSRGSSDPQGVTCGAATLSAERVRGDNKEYEYSVECQ
EDSACPAAEESLPIEVMVDAVHKLKYENYTSSFFIRDIIKPDPPKNLQLKPLKNSRQVEVS
WEYPDTWSTPHSYFSLTFCVQVQGKSKREKKDRVFTDKTSATVICRKNASISVRAQDRY
YSSSWSEWASVPCSGGGGSGGGGSGGGGSRNLPVATPDPGMFPCLHHSQNLLRAVSNML
QKARQTLEFYPCTSEEIDHEDITKDKTSTVEACLPLELTKNESCLNSRETSFITNGSCLASR
KTSFMMALCLSSIYEDLKMYQVEFKTMNAKLLMDPKRQIFLDQNMLAVIDELMQALNF
NSETVPQKSSLEEPDFYKTKIKLCILLHAFRIRAVTIDRVMSYLNAS

The amino acid sequence of Nanoluc is set forth in SEQ ID NO. 7: 
VFTLEDFVGDWRQTAGYNLDQVLEQGGVSSLFQNLGVSVTPIQRIVLSGENGL
KIDIHVIIPYEGLSGDQMGQIEKIFKVVYPVDDHHFKVILHYGTLVIDGVTPNMIDYFGRP
YEGIAVFDGKKITVTGTLWNGNKIIDERLINPDGSLLFRVTINGVTGWRLCER
2) All these plasmids were transfected into Expi293 cells or HEK293F cells. After transfection, the cells were cultured for 72 h, then centrifuged at 5000 rpm for 30 min to collect the cell supernatant, and the EVs were purified.
3) Loading verification: The cell positivity rate was assessed using FITC-scIL12 antibody labeling, and the Nanoluc enzyme activity in the EVs was measured to compare the efficiency of loading protein onto EVs membrane for each transmembrane domain.

### Results:

The cell positivity rates and EVs loading efficiencies for Nanoluc with TMD_Mut_10, TMD_Mut_13, TMD_Mut_17, and NPTN-TMD were significantly increased compared to EWI-F-TMD, as evidenced by higher cell positivity rates, greater single-cell intensity, and higher enzyme activity per EV particle compared to the wild-type EWI-F/EWI-2-TMD. **Therefore, in the subsequent plasmid construction, these four transmembrane domains were selected**, with sequences as follows:
The amino acid sequence of TMD_Mut_10 (G6N, S8L, L14W, S15L, C16F, G19L, S23L) is set forth in SEQ ID NO. 5:
   **LLIGVNLLTVIGLWLFLILYCSL**
The amino acid sequence of TMD_Mut_13 (S8L, S15L, C16F, G19L) is set forth in SEQ ID NO. 8:
   **LLIGVGLLTVIGLLLFLILYCSS**
The amino acid sequence of TMD_Mut_17 (G19S) is SEQ ID NO. 9:
   **LLIGVGLSTVIGLLSCLISYCSS**
The amino acid sequence of NPTN-TMD is SEQ ID NO. 10:
   LWPFLGILAEIIILVVIIVVVY

**Table 2 Comparison of Loading Efficiency for Different TMDs**

| No. | Cell Positivity Rate after scIL12 Antibody Labeling | Mean Fluorescence Intensity (MFI) after scIL12 Antibody Labeling | Nanoluc Enzyme Activity in EVs (per 1E+09 particles) |
|---|---|---|---|
| TMD_Mut_1 | 53.20% | 2758 | 1.93E+05 |
| TMD_Mut_2 | 31.88% | 4632 | 2.41E+05 |
| TMD_Mut_3 | 94.02% | 21226 | 1.85E+05 |
| TMD_Mut_4 | 50.00% | 8245 | 1.29E+05 |
| TMD_Mut_5 | 92.60% | 21905 | 2.05E+05 |
| TMD_Mut_6 | 75.68% | 11883 | 2.04E+05 |
| TMD_Mut_7 | 2.76% | 10589 | 2.29E+05 |
| TMD_Mut_8 | 4.76% | 9995 | 1.93E+05 |
| TMD_Mut_9 | 94.69% | 16938 | 2.15E+05 |
| TMD_Mut_10 | 96.63% | 39503 | 2.31E+05 |
| TMD_Mut_11 | 92.87% | 17127 | 2.33E+05 |
| TMD_Mut_12 | 18.25% | 10899 | 2.21E+05 |
| TMD_Mut_13 | 97.57% | 39460 | 2.11E+05 |
| TMD_Mut_14 | 96.52% | 27268 | 1.98E+05 |
| TMD_Mut_15 | 11.43% | 7362 | 1.97E+05 |
| TMD_Mut_16 | 96.13% | 16606 | 2.21E+05 |
| TMD_Mut_17 | 96.89% | 24486 | 2.21E+05 |
| TMD_Mut_18 | 82.98% | 4284 | 2.25E+05 |
| TMD_Mut_19 | 20.83% | 4737 | 2.14E+05 |
| TMD_Mut_20 | 77.02% | 4391 | 1.76E+05 |
| EWI-2-TMD (Wild-type EWI-2 Transmembrane Domain Sequence) | 88.1% | 10225 | 1.87E+05 |
| EWI-F-TMD (Wild-type EWI-F Transmembrane Domain Sequence) | 90.69% | 19952 | 1.39E+05 |
| NPTN-TMD (Wild-type NPTN Transmembrane Domain Sequence) | **97.51 %** | 41908 | 2.20E+05 |

### Example 2: Sequence Design and Validation of Tail Module

Objective: Designing a fluorescent protein or chemiluminescence module and loading them onto EVs membrane for detecting and tracing the successfully loaded EVs.

Principle: Plasmids containing a C-terminal detection module were constructed and transfected into HEK293F cells, and the engineering loaded EVs were purified. Three proteins were designed: green fluorescent protein EGFP, chemiluminescent protein Nanoluc, and Rluc.

### 2.1. EGFP

### Steps:

1) The plasmid, phCD24-Fc-TMD(NPTN-TMD)-ICD(EWI-F-ICD)-EGFP was constructed with pHEK293_Ultra_Expression_I (Takara) as the vector.

The amino acid sequence of EGFP is set forth in SEQ ID NO. 11:

MVSKGEELFTGVVPILVELDGDVNGHKFSVSGEGEGDATYGKLTLKFICTTGKL
PVPWPTLVTTLTYGVQCFSRYPDHMKQHDFFKSAMPEGYVQERTIFFKDDGNYKTRAE
VKFEGDTLVNRIELKGIDFKEDGNILGHKLEYNYNSHNVYIMADKQKNGIKVNFKIRHNI
EDGSVQLADHYQQNTPIGDGPVLLPDNHYLSTQSALSKDPNEKRDHMVLLEFVTAAGIT
LGMDELYK

The amino acid sequence of Fc is set forth in SEQ ID NO. 12:

AESKYGPPCPPCPAPEAAGGPSVFLFPPKPKDTLMISRTPEVTCVVVDVSQEDPE
VQFNWYVDGVEVHNAKTKPREEQFNSTYRVVSVLTVLHQDWLNGKEYKCKVSNKGLP
SSIEKTISKAKGQPREPQVYTLPPSQEEMTKNQVSLTCLVKGFYPSDIAVEWESNGQPEN
NYKTTPPVLDSDGSFFLYSRLTVDKSRWQEGNVFSCSVMHEALHNHYTQKSLSLSLG

The amino acid sequence of hCD24 is set forth in SEQ ID NO. 13: 
MGRAMVARLGLGLLLLALLLPTQIYSSETTTGTSSNSSQSTSNSGLAPNPTNATT
KAAG
2) The plasmid was transfected into HEK293F cells. After transfection, the cells were cultured for 72 h, then centrifuged at 5000 rpm for 30 min to collect the cell supernatant, and EVs were purified from the supernatant.
3) Loading verification: After 72 h post-transfection, 2E+05 cells were collected, washed twice with 1×PBS, and then resuspended in 200 µl 1×PBS. Flow cytometry was used to detect EGFP expression at the cellular level. The purified EVs were analyzed using NanoFCM (Xiamen Fuliu) to detect the loading of EGFP on the EVs membrane.

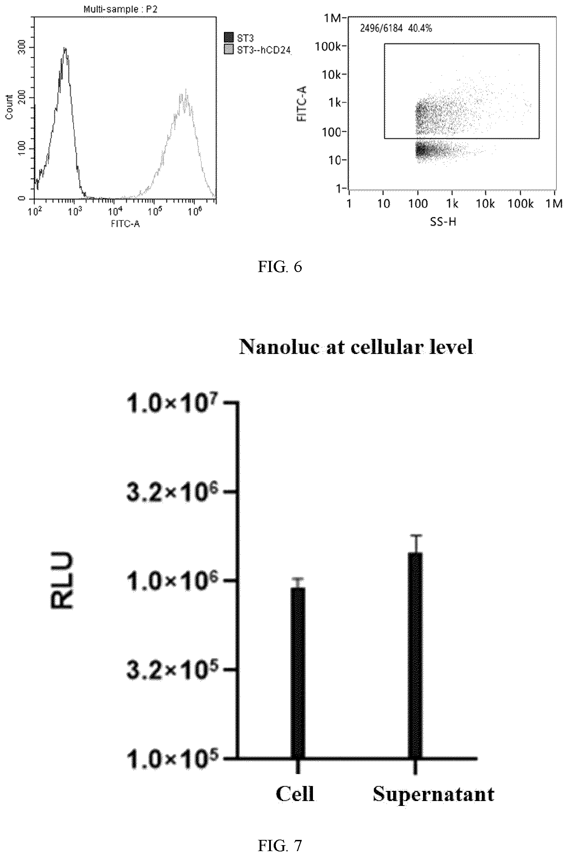

The results are shown in FIG. 6:
The positivity rate for EGFP expression in HEK293F cells after transfection reached 98.91%, and the percentage of EVs loaded with EGFP after purification was about 40.4%.

### 2.2. Nanoluc

### Steps:

1) The plasmid, pscIL12-TMD(TMD_Mut_17)-ICD(EWI-F-ICD)-Nanoluc was constructed with pHEK293_Ultra_Expression_I (Takara) as the vector.
   The amino acid sequence of Nanoluc is set forth in SEQ ID NO. 7: 
VFTLEDFVGDWRQTAGYNLDQVLEQGGVSSLFQNLGVSVTPIQRIVLSGENGL
KIDIHVIIPYEGLSGDQMGQIEKIFKVVYPVDDHHFKVILHYGTLVIDGVTPNMIDYFGRP
YEGIAVFDGKKITVTGTLWNGNKIIDERLINPDGSLLFRVTINGVTGWRLCER
2) The plasmid was transfected into HEK293F cells. After transfection, the cells were cultured for 72 h, then centrifuged at 5000 rpm for 30 min to collect the cell supernatant, and EVs were purified from the supernatant.
3) Loading verification: After 72 h post-transfection, 1E+03 cells were collected, washed twice with 1×PBS, and then resuspended in 50 µl 1×PBS. A microplate reader was used to measure the Nanoluc enzyme activity signal in the cells, indicating Nanoluc protein expression in the cells. The enzyme activity signal in the purified EVs was similarly measured by a microplate reader to indicate the loading density of Nanoluc on the EVs membrane.

The results are shown in FIG. 7.

At the cellular level, the enzyme activity signal in the supernatant ranged from 1E+06 to 2E+06 RLU, equivalent to an enzyme activity signal of 8E+05 to 1.0E+06.per 10³ cells.

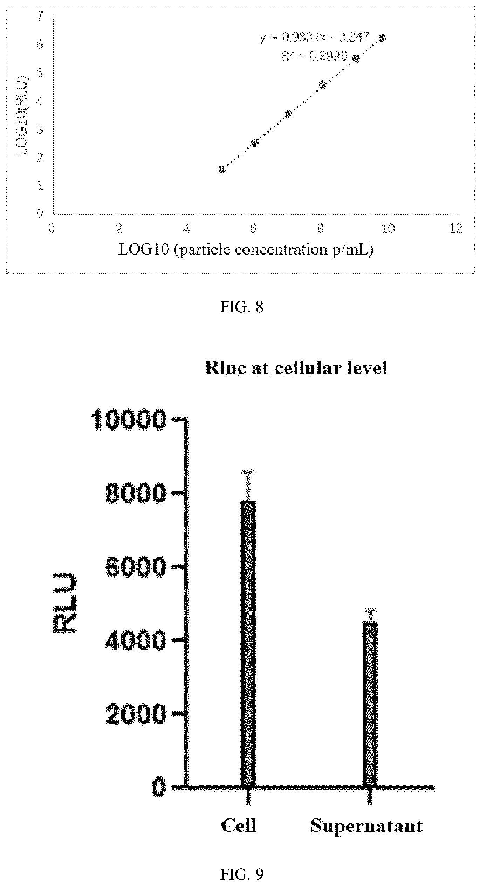

The purified EVs exhibited an enzyme activity of about 1.06E+05 RLU per 10⁹ EVs particles, and the enzyme activity showed a linear relationship with the number of EVs particles. The standard curve is shown in FIG. 8.

### 2.3 Rluc

### Steps:

1) The plasmid, pscIL12-TMD(TMD_Mut_10)-ICD(EWI-F-ICD)-Rluc was constructed with pHEK293_Ultra_Expression_I (Takara) as the vector.
   The amino acid sequence of Rluc is set forth in SEQ ID NO. 14: 
ASKVYDPEQRKRMITGPQWWARCKQMNVLDSFINYYDSEKHAENAVIFLHGN
AASSYLWRHVVPHIEPVARCIIPDLIGMGKSGKSGNGSYRLLDHYKYLTAWFELLNLPK
KIIFVGHDWGACLAFHYSYEHQDKIKAIVHAESVVDVIESWDEWPDIEEDIALIKSEEGEK
MVLENNFFVETMLPSKIMRKLEPEEFAAYLEPFKEKGEVRRPTLSWPREIPLVKGGKPDV
VQIVRNYNAYLRASDDLPKMFIESDPGFFSNAIVEGAKKFPNTEFVKVKGLHFSQEDAPD
EMGKYIKSFVERVLKNEQ
2) The plasmid was transfected into HEK293F cells. After transfection, the cells were cultured for 72 h, then centrifuged at 5000 rpm for 30 min to collect the cell supernatant, and EVs were purified from the supernatant.
3) Loading verification: After 72 h post-transfection, 1E+03 cells were collected, washed twice with 1×PBS, and then resuspended in 50 µl 1×PBS. A microplate reader was used to measure the Rluc enzyme activity signal in the cells, indicating Rluc protein expression in the cells. The enzyme activity signal in the purified EVs was similarly measured by a microplate reader to indicate the loading density of Rluc on the EVs membrane.

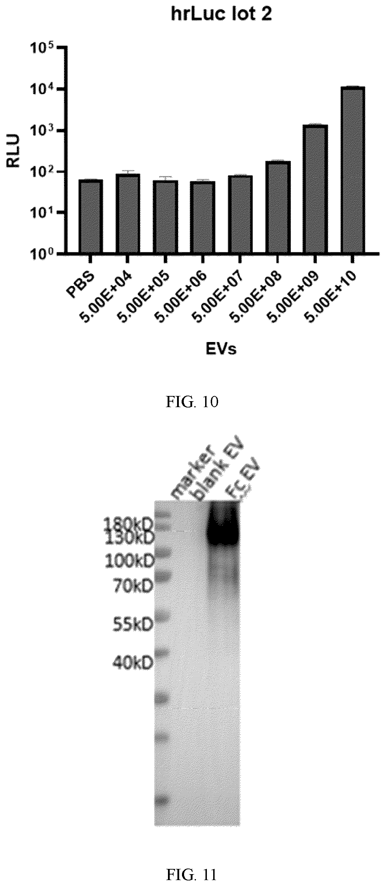

The results are shown in FIG. 9 and FIG. 10.

The enzyme activity signal in the cell supernatant ranged from 4E+03 and 5E+03 RLU, equivalent to an enzyme activity signal of 7E+03 and 9E+03 RLU per 10³ cells.

The purified EVs exhibited an enzyme activity of about 300-500 RLU per 10⁹ EVs particles, and the enzyme activity showed a linear relationship within the range of 10⁸-10¹⁰ EVs particles, with lower concentrations of EVs showing enzyme activity below the detection limit. Rluc loading and detection were less sensitive compared to Nanoluc.

### Example 3: Design and Verification of Scaffold Domain Sequence

### Objective: 4 different scaffold sequences:

a. Fc, which uses intermolecular disulfide bonds to form dimers, with good fusion properties and resistance to degradation, and is used for EV purification by affinity chromatography.
   The amino acid sequence of Fc is set forth in SEQ ID NO. 12: 
AESKYGPPCPPCPAPEAAGGPSVFLFPPKPKDTLMISRTPEVTCVVVDVSQEDPE
VQFNWYVDGVEVHNAKTKPREEQFNSTYRVVSVLTVLHQDWLNGKEYKCKVSNKGLP
SSIEKTISKAKGQPREPQVYTLPPSQEEMTKNQVSLTCLVKGFYPSDIAVEWESNGQPEN
NYKTTPPVLDSDGSFFLYSRLTVDKSRWQEGNVFSCSVMHEALHNHYTQKSLSLSLG
b. Fc+NPTN-Ig1-3, with NPTN-Ig1-3 added to the C-terminus of Fc to form dimers, extending the scaffold and reducing steric hindrance for membrane protein loading, thereby increasing the chances of protein loading onto the EVs membrane.
   The amino acid sequence of Fc+NPTN-Ig1-3 is set forth in SEQ ID NO. 15: 
AESKYGPPCPPCPAPEAAGGPSVFLFPPKPKDTLMISRTPEVTCVVVDVSQEDPEVQFNW
YVDGVEVHNAKTKPREEQFNSTYRVVSVLTVLHQDWLNGKEYKCKVSNKGLPSSIEKTI
SKAKGQPREPQVYTLPPSQEEMTKNQVSLTCLVKGFYPSDIAVEWESNGQPENNYKTTP
 
PVLDSDGSFFLYSRLTVDKSRWQEGNVFSCSVMHEALHNHYTQKSLSLSLGQNAGFVKS
PMSETKLTGDAFELYCDVVGSPTPEIQWWYAEVNRAESFRQLWDGARKRRVTVNTAY
GSNGVSVLRITRLTLEDSGTYECRASNDPKRNDLRQNPSITPRIVTSEEVIIRDSPVLPVTL
QCNLTSSSHTLTYSYWTKNGVELSATRKNASNMEYRINKPRAEDSGEYHCVYHFVSAPK
ANATIEVKPDITGHKRSENKNEGQDATMYCKSVGYPHPDWIWRKKENGMPMDIVNTSG
RFFIINKENYTELNIVNLQITEDPGEYECNATNAIGSASVVT
c. ITGB1-I-EGF1-4: with ITGB1 knocked out in background cells and overexpressing a transmembrane protein containing ITGB1-I-EGF1-4 to increase protein loading density.
   The amino acid sequence of ITGB1-I-EGF1-4 is set forth in SEQ ID NO. 16: 
CQSEGIPESPKCHEGNGTFECGACRCNEGRVGRHCECSTDEVNSEDMDAYCRK
ENSSEICSNNGECVCGQCVCRKRDNTNEIYSGKFCECDNFNCDRSNGLICGGNGVCKCR
VCECNPNYTGSACDCSLDTSTCEASNGQICNGRGICECGVCKCTDPKFQGQTCEMCQT
d. Foldon+NPTN-Ig1-3, with Foldon sequence added to the N-terminus of the long scaffold sequence NPTN-Ig1-3 to form a trimeric structure, increasing the local concentration of effector proteins at the target site.

The amino acid sequence of Foldon+NPTN-Ig1-3 is set forth in SEQ ID NO. 17:

YIPEAPRDGQAYVRKDGEWVLLSTFLGQNAGFVKSPMSETKLTGDAFELYCDV
VGSPTPEIQWWYAEVNRAESFRQLWDGARKRRVTVNTAYGSNGVSVLRITRLTLEDSG
TYECRASNDPKRNDLRQNPSITPRIVTSEEVIIRDSPVLPVTLQCNLTSSSHTLTYSYWTKN
GVELSATRKNASNMEYRINKPRAEDSGEYHCVYHFVSAPKANATIEVKPDITGHKRSEN
KNEGQDATMYCKSVGYPHPDWIWRKKENGMPMDIVNTSGRFFIINKENYTELNIVNLQI
TEDPGEYECNATNAIGSASVVT

Principle: Plasmids containing different scaffold sequences were constructed, transfected into HEK293F cells, and the supernatant from cell culture was purified to obtain the engineering loaded EVs.

### 3.1 Fc

### Steps:

1) The plasmid, phCD24-Fc-TMD(NPTN-TMD)-ICD(EWI-2-ICD)-EGFP was constructed with pHEK293_Ultra_Expression_I (Takara) as the vector.
2) The plasmid was transfected into HEK293F cells. After transfection, the cells were cultured for 72 h, then centrifuged at 5000 rpm for 30 min to collect the cell supernatant, and crude EVs were purified from the supernatant.
3) Loading Verification: EVs were analyzed by Western Blot using an Fc antibody to verify the Fc loading in EVs. Protein A affinity chromatography was performed by equilibrating a Protein A column with 1×PBS for three column volumes (CV). After applying the crude EVs sample, the column was further equilibrated with 1×PBS for three CVs. Elution was performed with 50 mM sodium citrate pH 3.0 for three CVs. NanoFCM was used to detect EVs particle count and positivity rate, confirming that Protein A could enrich Fc-tagged EVs.

The results are shown in FIG. 11.

The theoretical molecular weight of hCD24-Fc-TMD(NPTN-TMD)-ICD(EWI-2-ICD)-EGFP is 102 kD. Western Blot results of the Fc antibody confirmed the loading of Fc.

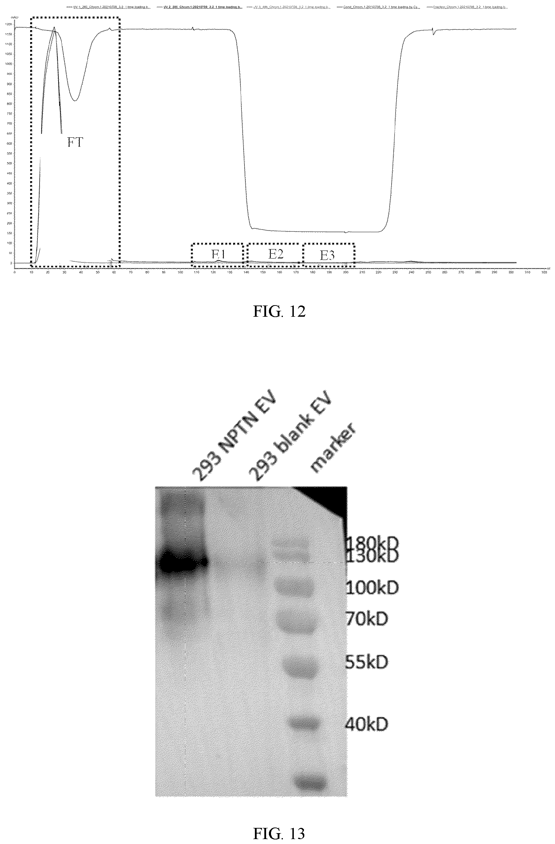

The positivity rate of EVs was 10.7% before Protein-A resin purification and increased to 67.9% after Protein-A resin purification. "Before binding" refers to the sample before purification, "FT" denotes the flow-through fraction from the Protein A column, and "E1," "E2," and "E3" represent the three elution fractions. These results demonstrate that Protein A is effective in enriching Fc-tagged EVs, with the majority of Fc-tagged EVs being found in the E1 and E2 fractions. The results are shown in FIG. 12 and Table 3.

**Table 3 NanoFCM Detection Results for Protein A-Purified EVs**

| Sample Name | Elution Volume (mL) | Elution Particle Concentration (Particles/mL) | Positivity Rate (%) |
|---|---|---|---|
| before binding | 10 | 4.69E+11 | 10.70 |
| FT | 15 | 1. 30E+10 | 2.30 |
| E1 | 2 | 3.53E+10 | 53.20 |
| E2 | 2 | 2.61E+10 | 67.90 |
| E3 | 2 | 1.41E+9 | 14.90 |

### 3.2 Fc+NPTN-Ig1-3

### Steps:

1) The plasmid, phCD24-Fc-NPTN-Ig1-3-TMD(NPTN-TMD)-ICD(EWI-2-ICD)-EGFP was constructed with pHEK293_Ultra_Expression_I (Takara) as the vector.
   The amino acid sequence of hCD24 is set forth in SEQ ID NO. 13: 
MGRAMVARLGLGLLLLALLLPTQIYSSETTTGTSSNSSQSTSNSGLAPNPTNATT
KAAG
2) The plasmid was transfected into HEK293F cells. After transfection, the cells were cultured for 72 h, then centrifuged at 5000 rpm for 30 min to collect the cell supernatant, and EVs were purified from the supernatant.
3) Loading Verification: EVs were analyzed by Western Blot using an NPTN antibody to verify the loading of NPTN-Ig1-3 onto the EVs.

The results are shown in FIG. 13.

The theoretical molecular weight of hCD24-Fc-NPTN-Ig1-3-TMD(NPTN-TMD)-ICD(EWI-2-ICD)-EGFP is 102 kD. Western Blot results of the NPTN antibody confirmed the loading of NPTN-Ig1-3.

### 3.3 ITGB1-I-EGF1-4 Sequence

### Steps:

1) Using as a vector, The plasmid, pCD47-ITGB1-I-EGF1-4-TMD(TMD_Mut_10)-ICD(EWI-F-ICD)-Nanoluc was constructed with pHEK293_Ultra_Expression_I (Takara) as the vector.
   The amino acid sequence of is CD47 shown in SEQ ID NO. 18: 
QLLFNKTKSVEFTFCNDTVVIPCFVTNMEAQNTTEVYVKWKFKGRDIYTFDGA
LNKSTVPTDFSSAKIEVSQLLKGDASLKMDKSDAVSHTGNYTCEVTELTREGETIIELKY
RVVSW
2) The plasmid was transfected into HEK293F cells. After transfection, the cells were cultured for 72 h, then centrifuged at 5000 rpm for 30 min to collect the cell supernatant, and EVs were purified from the supernatant.
3) Loading Verification: EVs were analyzed by Western Blot using an ITGB1 antibody to verify the loading of ITGB1 onto the EVs

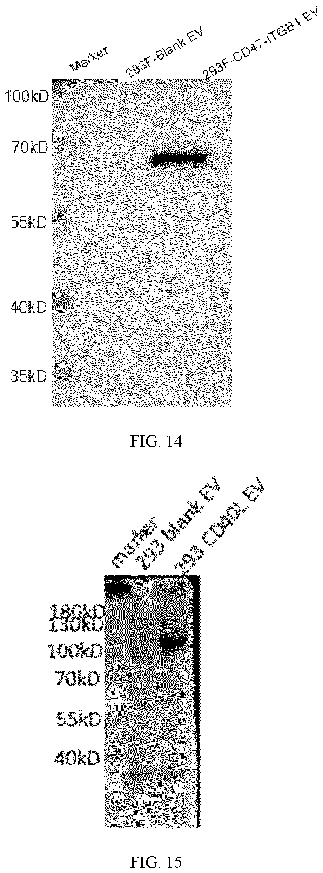

The results are shown in FIG. 14.

The theoretical molecular weight of CD47-ITGB1-I-EGF1-4-TMD(TMD_Mut_10)-ICD(EWI-F-ICD)-Nanoluc is 65 kD. Western Blot results of the ITGB1 antibody confirmed the loading of ITGB1.

### 3.4 Foldon+NPTN-Ig1-3

### Steps:

1) The plasmid, pCD40L-Foldon-NPTN-Ig1-3-TMD(NPTN-TMD)-ICD(EWI-F-ICD)-Nanoluc was constructed with pHEK293_Ultra_Expression_I (Takara) as the vector.
   The amino acid sequence of CD40L is set forth in SEQ ID NO. 19: 
GDQNPQIAAHVISEASSKTTSVLQWAEKGYYTMSNNLVTLENGKQLTVKRQGL
YYIYAQVTFCSNREASSQAPFIASLCLKSPGRFERILLRAANTHSSAKPCGQQSIHLGGVF
ELQPGASVFVNVTDPSQVSHGTGFTSFGLLKL
2) The plasmid was transfected into HEK293F cells. After transfection, the cells were cultured for 72 h, then centrifuged at 5000 rpm for 30 min to collect the cell supernatant, and EVs were purified from the supernatant.
3) Loading Verification: EVs were analyzed by Western Blot using an CD40L antibody to verify the loading of CD40L onto the EVs.

The results are shown in FIG. 15.

The theoretical molecular weight of CD40L-Foldon-NPTN-Ig1-3-TMD (NPTN-TMD)-ICD (EWI-F-ICD)-Nanoluc is 104 kD (with Foldon forming trimers with CD40L). Western Blot results of the CD40L antibody confirmed the loading of CD40L (trimer).

### Example 4: Design of Intracellular Domain (ICD) Sequences

Objective: Anchoring loaded proteins onto the membrane using s-palmitoyl cysteine in the ICD sequences of EWI-F and EWI-2.

Principle: The ICD sequences of EWI-F and EWI-2 were used to construct all the plasmids. The plasmids were transfected into HEK293F cells, and the cell culture supernatant was purified to obtain the engineering loaded EVs.

The amino acid sequence of EWI-F-ICD is set forth in SEQ ID NO. 25: HWCCKKEVQETRRERRRLMSMEMD

The amino acid sequence of EWI-2-ICD is set forth in SEQ ID NO. 26: TITCCFMKRLRKR

Results: The loaded sequences were all detected on EVs, proving the anchoring effect of EWI-F-ICD and EWI-2-ICD.

### Example 5: Design and Verification of Extracellular Functional Protein Modules (Head) Sequences

Objective: Loading extracellular effector proteins to target specific cells or exert direct therapeutic effects.

Principle: Plasmids containing sequences of different functional proteins were constructed, and transfected into HEK293F cells. The cell culture supernatant was purified to obtain engineering loaded EVs, and the loading and efficacy of different proteins were verified on the basis of their functions.

### 5.1 APOE

1) The plasmid, pAPOE-NPTN-Ig1-3-TMD(NPTN-TMD)-ICD (EWI-2-ICD) was constructed with pHEK293_Ultra_Expression_I (Takara) as the vector.
   The amino acid sequence of APOE is set forth in SEQ ID NO. 20: 
KVEQAVETEPEPELRQQTEWQSGQRWELALGRFWDYLRWVQTLSEQVQEELL
SSQVTQELRALMDETMKELKAYKSELEEQLTPVAEETRARLSKELQAAQARLGADMED
VCGRLVQYRGEVQAMLGQSTEELRVRLASHLRKLRKRLLRDADDLQKRLAVYQAGARE
GAERGLSAIRERLGPLVEQGRVRAATVGSLAGQPLQERAQAWGERLRARMEEMGSRTR
DRLDEVKEQVAEVRAKLEEQAQQIRLQAEAFQARLKSWFEPLVEDMQRQWAGLVEKVQ
AAVGTSAAPVPSDNH
2) The plasmid was transfected into HEK293F cells. After transfection, the cells were cultured for 72 h, then centrifuged at 5000 rpm for 30 min to collect the cell supernatant, and EVs were purified from the supernatant.
3) Loading Verification: The binding of APOE-loaded EVs to the low-density lipoprotein receptor-related protein (LRP) was detected using Biacore-T200 (Cytiva). This demonstrated the ability of APOE-loaded EVs to bind to receptor proteins. A Protein A chip was used with LRP as the stationary phase and PBST as the buffer. APOE-loaded EVs served as the mobile phase, with a flow rate of 10 µL/min.

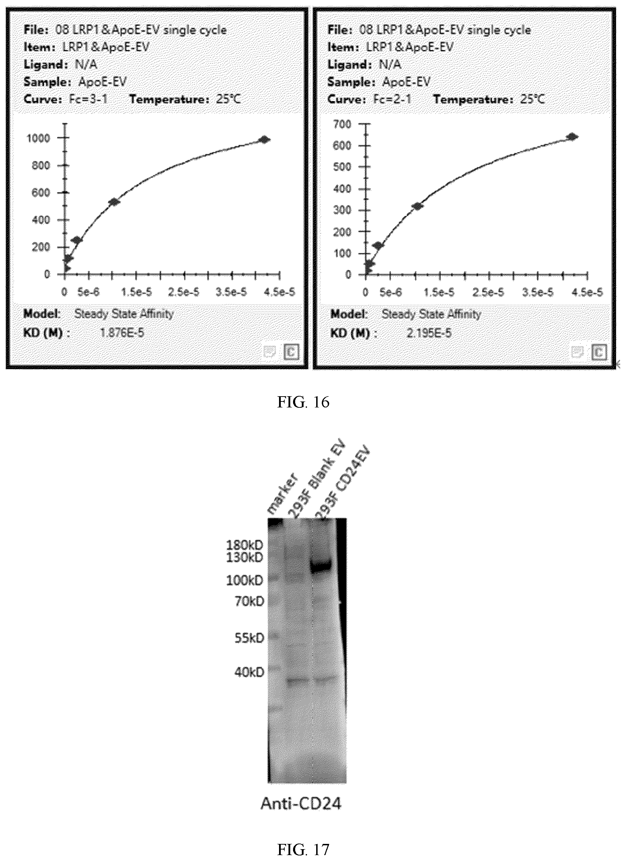

The results are shown in FIG. 16.

The binding of APOE-loaded EVs to LRP was detected, with an equilibrium dissociation constant of approximately 2×10⁻⁵ mol/L.

### 5.2 hCD 24

### Steps:

1) The plasmid, phCD24-Fc-TMD(NPTN-TMD)-ICD(EWI-2-ICD)-EGFP was constructed with pHEK293_Ultra_Expression_I (Takara) as the vector.
2) The plasmid was transfected into HEK293F cells. After transfection, the cells were cultured for 72 h, then centrifuged at 5000 rpm for 30 min to collect the cell supernatant, and EVs were purified from the supernatant.
3) Loading Verification: Western Blot analysis was performed on EVs using an hCD24 antibody to verify the loading of hCD24 onto the EVs.
4) Efficacy Verification

Human PBMC cells were treated with varying doses of EVs loaded with full-length hCD24-Fc-TMD(NPTN-TMD)-ICD(EWI-2-ICD)-EGFP. The supernatant was collected, and ELISA was used to measure the levels of IFN-γ secretion by PBMC cells in each treatment group.

The results are shown in FIGs. 17-18:
1) Loading Verification: The theoretical molecular weight of hCD24-Fc-TMD(NPTN-TMD)-ICD(EWI-2-ICD)-EGFP is 82.5 kD. Western Blot results of the hCD24 antibody confirmed the loading of hCD24, with the observed molecular weight being higher due to glycosylation modifications of hCD24 itself.
2) Efficacy Verification: hCD24-loaded EVs can significantly reduce IFN-γ secretion from PBMC stimulated by CD3 and CD28 antibodies, with a significant dose-dependent inhibition. The inhibitory effect increased with the higher number of EVs particles. The effect of 10⁷ EVs was approximately equivalent to 10 µg/mL (2.4 µg) of hCD24 protein.

### 5.3 ScFv

### 5.3.1 Anti-CD3-ScFv(L-H)

### Steps:

1) The plasmid, pAnti-CD3-ScFv(L-H)-NPTN-Ig1-3-TMD(TMD_Mut_17)-ICD(EWI-2-ICD)-EGFP was constructed with pHEK293_Ultra_Expression_I (Takara) as the vector.
   The amino acid sequence of Anti-CD3-ScFv(L-H) is set forth in SEQ ID NO. 21: 
QTVVTQEPSLTVSPGGTVTLTCGSSTGAVTSGNYPNWVQQKPGQAPRGLIGGTK
FLAPGTPARFSGSLLGGKAALTLSGVQPEDEAEYYCVLWYSNRWVFGGGTKLTVLGGGG
SGGGGSGGGGSEVQLVESGGGLVQPGGSLKLSCAASGFTFNKYAMNWVRQAPGKGLE
WVARIRSKYNNYATYYADSVKDRFTISRDDSKNTAYLQMNNLKTEDTAVYYCVRHGNFG
NSYISYWAYWGQGTLVTVSS
2) The plasmid was transfected into HEK293F cells. After transfection, the cells were cultured for 72 h, then centrifuged at 5000 rpm for 30 min to collect the cell supernatant, and EVs were purified from the supernatant.
3) Loading Verification
   A. NanoFCM was used to detect the expression of EGFP on EVs.
   B. EVs were analyzed by Western Blot using an Anti-CD3-ScFv antibody to verify the loading of Anti-CD3-ScFv onto the EVs.
4) Efficacy Verification:
   Human PBMC cells were stimulated using EVs loaded with Anti-CD3-ScFv(L-H)-NPTN-Ig1-3-TMD(TMD_Mut_17)-ICD(EWI-2-ICD)-EGFP, combined with 1 µg/mL anti-CD28 antibody, and the activation of PBMC by the EVs was detected.

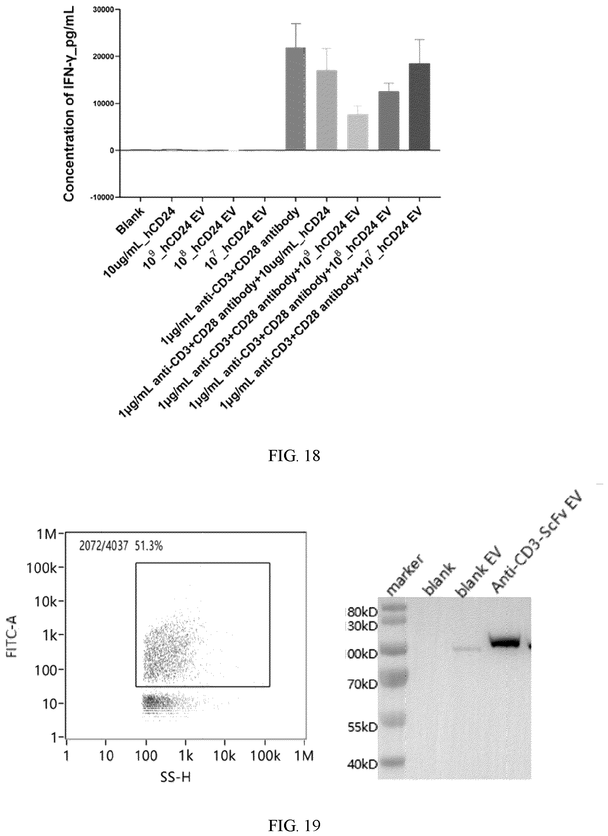

The results are shown in FIGs. 19-20.
1) Loading Verification:
   A. NanoFCM results showed that the positivity rate of EVs loaded with EGFP reached 51.3%.
   B. The theoretical molecular weight of Anti-CD3-ScFv(L-H)-NPTN-Ig1-3-TMD(TMD_Mut_17)-ICD(EWI-2-ICD)-EGFP is 97 kD. Western Blot results of the Anti-CD3-ScFv antibody confirmed the loading of Anti-CD3-ScFv(L-H).
2) Efficacy Verification:
   EVs loaded with Anti-CD3-ScFv(L-H)-NPTN-Ig1-3-TMD(TMD_Mut_17)-ICD(EWI-2-ICD)-EGFP combined with 1 µg/mL anti-CD28 antibody can activate human PBMCs and stimulate IFN-γ secretion in a significant dose-dependent manner. When the number of EVs particles exceeded 10⁷, the activation of PBMC was gradually enhanced with the increase in the number of EVs particles.

### 5.3.2 Anti-CLDN18.2-ScFv(L-H)

### Steps:

1) The plasmid, pAnti-CLDN18.2-ScFv(L-H)-Fc-TMD(TMD_Mut_17)-ICD(EWI-2-ICD)-Nanoluc was constructed with pHEK293_Ultra_Expression_I (Takara)as the vector.
   The amino acid sequence of Anti-CLDN18.2-ScFv(L-H) is set forth in SEQ ID NO. 22: 
DIQMTQSPSSLSASVGDRVTITCKSSQSLFNTGNQKNYLTWYQQKPGKAPKLLI
YWASTRESGVPSRFSGSGSGTDFTLTISSLQPEDFATYYCQNNYNFPLTFGQGTRLEIKGG
GGSGGGGSGGGGSQVQLVESGGGLVKPGGSLRLSCAASGFSFSSFGMHWVRQAPGKGL
EWVAYISSGSRTIYYADTVKGRFTISRDNAKNSLYLQMNSLRAEDTAVYYCTRYYYGNSF
DYWGQ GTTVTVSS
2) The plasmid was transfected into HEK293F cells. After transfection, the cells were cultured for 72 h, then centrifuged at 5000 rpm for 30 min to collect the cell supernatant, and EVs were purified from the supernatant.
3) Loading Verification: EVs were analyzed by Western Blot using an Anti-CLDN18.2-ScFv(L-H) antibody to verify the loading of Anti-CLDN18.2-ScFv(L-H) onto the EVs.
4) Efficacy Verification: The binding of EVs loaded with Anti-CLDN18.2-ScFv(L-H) to CLDN18.2-expressing HepG2 cells along with the ability of CLDN18.2 antibody to block this interaction was assessed to confirm the functional activity of EVs loaded with Anti-CLDN18.2-ScFv(L-H).

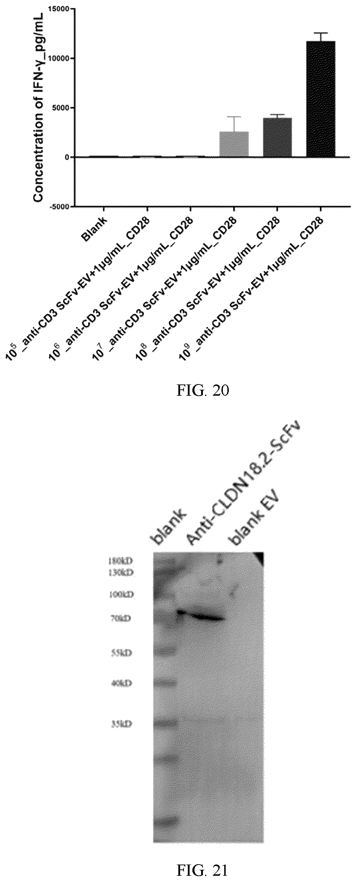

The results are shown in FIGs. 21-22:
1) Loading Verification:
   The theoretical molecular weight of Anti-CLDN18.2-ScFv(L-H)-Fc-TMD(TMD_Mut_17)-ICD(EWI-2-ICD)-Nanoluc is 83 kD. Western Blot results of the Anti-CLDN18.2-ScFv antibody confirmed the loading of Anti-CLDN18.2-ScFv(L-H).
2) Efficacy Verification: After treating CLDN18.2-expressing HepG2 cells with EVs loaded with Anti-CLDN18.2-ScFv(L-H), Nanoluc enzyme activity was detected in the HepG2 cells. Subsequent addition of CLDN18.2 antibody to the binding system resulted in a decrease in Nanoluc enzyme activity. This indicated that the Anti-CLDN18.2-ScFv(L-H) loaded on the EVs bound to the antigen protein on the HepG2 cell surface, and that this binding could be blocked by the CLDN18.2 antibody, thereby confirming the functional activity of the EVs loaded with Anti-CLDN18.2-ScFv(L-H).

### 5.3.3 Anti-GPC3-ScFv(L-H)

### Steps:

1) The plasmid, pAnti-GPC3-ScFv(L-H)-Fc-TMD(TMD_Mut_13)-ICD(EWI-2-ICD)-Nanoluc was constructed with pHEK293_Ultra_Expression_I (Takara)as the vector.
   The amino acid sequence of Anti-GPC3-ScFv(L-H) is set forth in SEQ ID NO. 23: 
DVVMTQSPLSLPVTPGEPASISCRSSQSLVHSNANTYLHWYLQKPGQSPQLLIYK
VSNRFSGVPDRFSGSGSGTDFTLKISRVEAEDVGVYYCSQNTHVPPTFGQGTKLEIKGGG
GSGGGGSGGGGSQVQLVQSGAEVKKPGASVKVSCKASGYTFTDYEMHWVRQAPGQGL
EWMGALDPKTGDTAYSQKFKGRVTLTADESTSTAYMELSSLRSEDTAVYYCTRFYSYTY
WGQGTLVTVSS
2) The plasmid was transfected into HEK293F cells. After transfection, the cells were cultured for 72 h, then centrifuged at 5000 rpm for 30 min to collect the cell supernatant, and EVs were purified from the supernatant.
3) Loading Verification:
   A. EVs were analyzed by Western Blot using an Anti-GPC3-ScFv(L-H) antibody to verify the loading of Anti-GPC3-ScFv(L-H) onto the EVs.
   B. The binding of EVs loaded with Anti-GPC3-ScFv(L-H) to the antigen protein was assessed using Fortebio's in vitro binding detection with an Fc sensor. GPC3-Fc antigen protein served as the stationary phase, and EVs acted as the mobile phase, with 1×PBS used as the wash buffer.

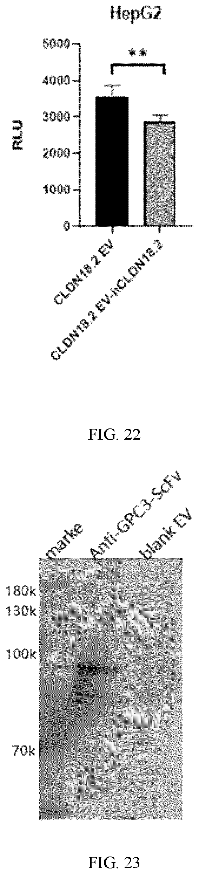

The results are shown in FIGs. 23-24 and Table 4.

### Loading Verification:

A. The theoretical molecular weight of Anti-GPC3-ScFv(L-H)-Fc-TMD(TMD_Mut_13)-ICD(EWI-2-ICD)-Nanoluc is 85 kD. Western Blot results of the Anti-GPC3-ScFv(L-H) antibody confirmed the loading of Anti-GPC3-ScFv(L-H).
B. The results from Fortebio indicated that the EVs loaded with Anti-GPC3-ScFv(L-H) bound to the antigen protein.

**Table 4. Affinity values of Anti-GPC3-ScFv(L-H) loaded EVs to the antigen**

| Raw | Sample ID | Response (nm) |
|---|---|---|
| A1 | ST3-Anti-GPC3-ScFv EVs | 0.5969 |
| B1 | ST3-Anti-GPC3-Fab | 0.3202 |
| C1 | ST3-Nanoluc EVs | 0.1374 |
| D1 | ST3-Blank EVs | 0.0427 |
| E1 | 1×PBS | Blank |

### 5.4 GDNF

### Steps:

1) The plasmid, pGDNF-Fc-TMD(NPTN-TMD)-ICD(EWI-2-ICD)-Nanoluc was constructed with pHEK293_Ultra_Expression_I (Takara) as the vector.
   The amino acid sequence of GDNF is set forth in SEQ ID NO. 24: 
MKLWDVVAVCLVLLHTASAFPLPAGKRPPEAPAEDRSLGRRRAPFALSSDSNMP
EDYPDQFDDVMDFIQATIKRLKRSPDKQMAVLPRRERNRQAAAANPENSRGKGRRGQR
GKNRGCVLTAIHLNVTDLGLGYETKEELIFRYCSGSCDAAETTYDKILKNLSRNRRLVSD
KVGQACCRPIAFDDDLSFLDDNLVYHILRKHSAKRCGCI
2) The plasmid was transfected into HEK293F cells. After transfection, the cells were cultured for 72 h, then centrifuged at 5000 rpm for 30 min to collect the cell supernatant, and EVs were purified from the supernatant.
3) Loading Verification: The enzyme activity levels of Nanoluc were measured using a Nanoluc substrate to verify the loading of GDNF onto EVs.

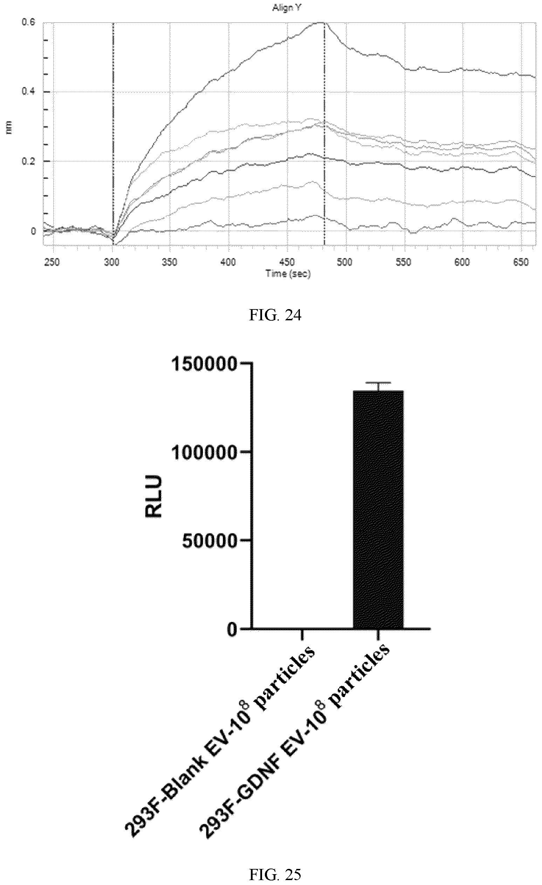

The results are shown in FIG. 25, indicating that EVs loaded with GDNF has higher enzyme activity levels of Nanoluc.

### 5.5 CD40L

### Steps:

1) The plasmid, pCD40L-Foldon-NPTN-Ig1-3-TMD(NPTN-TMD)-ICD(EWI-2-ICD)-Nanoluc was constructed with pHEK293_Ultra_Expression_I (Takara) as the vector.
2) The plasmid was transfected into HEK293F cells. After transfection, the cells were cultured for 72 h, then centrifuged at 5000 rpm for 30 min to collect the cell supernatant, and EVs were purified from the supernatant.
3) Loading Verification
   A. The constructed plasmid was first transfected into HEK293F cells. Flow cytometry analysis was performed using APC-anti CD40L antibody to assess the expression of the plasmid at the cellular level.
   B. The enzyme activity levels of Nanoluc were measured using a Nanoluc substrate to verify the loading of CD40L onto EVs.
4) Efficacy Verification: EVs loaded with CD40L-Foldon-NPTN-Ig1-3-TMD(NPTN-TMD)-ICD(EWI-2-ICD)-Nanoluc were used to treat B cells, and then the activation of B cells by the EVs was detected.

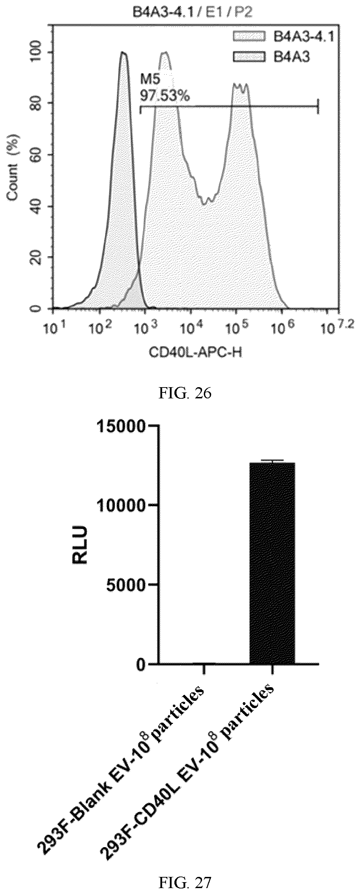

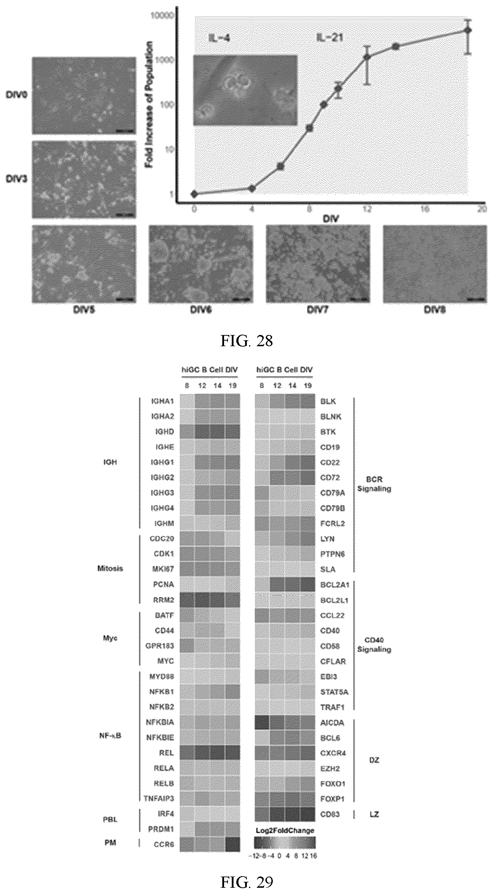

The results are shown in FIGs. 26-29.

### 1) Loading Verification:

A. Flow cytometry results indicated high expression levels of CD40L at the cellular level following plasmid transfection.
B. EVs loaded with CD40L had a high level of Nanoluc enzyme activity.

### 2) Efficacy Verification

A. EVs loaded with CD40L can promote B cell proliferation;
B. EVs loaded with CD40L can increase the expression of B cell activation markers.

### 5.6 CD47

### Steps:

1) The plasmid, pCD47-ITGB1-I-EGF1-4-TMD(TMD_Mut_10)-ICD(EWI-F-ICD)-Nanoluc was constructed with pHEK293_Ultra_Expression_I (Takara) as the vector.
2) The plasmid was transfected into HEK293F cells. After transfection, the cells were cultured for 72 h, then centrifuged at 5000 rpm for 30 min to collect the cell supernatant, and EVs were purified from the supernatant.
3) Loading Verification:
   A. After co-incubation of EVs loaded with CD47 with GFP-anti-CD47 antibody, free antibodies were removed through core700, and NanoFCM was used to detect GFP positivity in the samples.
   B. The enzyme activity levels of Nanoluc were measured using a Nanoluc substrate to verify the loading of CD47 onto EVs.

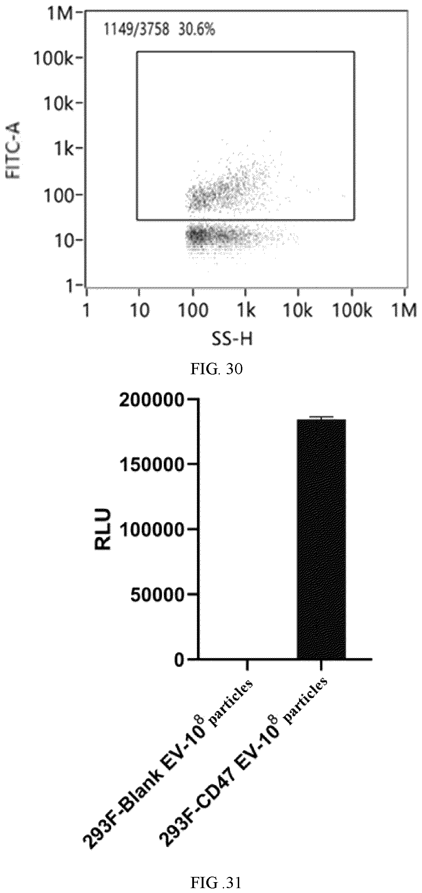

The results are shown in FIGs. 30-31:

### Loading Verification:

A. The NanoFCM analysis showed approximately 30% GFP positivity in the samples, indicating successful loading of CD47 onto the EVs.
B. EVs loaded with CD47 had a high level of Nanoluc enzyme activity.

**5.7 scIL12**

### Steps:

1) The plasmid, pscIL12-NPTN-Ig1-3-TMD(NPTN-TMD)-ICD(EWI-F-ICD)-Nanoluc was constructed with pHEK293_Ultra_Expression_I (Takara) as the vector.
2) The plasmid was transfected into HEK293F cells. After transfection, the cells were cultured for 72 h, then centrifuged at 5000 rpm for 30 min to collect the cell supernatant, and EVs were purified from the supernatant.
3) Loading Verification: The enzyme activity levels of Nanoluc were measured using a Nanoluc substrate to verify the loading of scIL onto EVs.
4) Efficacy Verification:

EVs loaded with scIL-12-NPTN-Ig1-3-TMD(NPTN-TMD)-ICD (EWI-F-ICD)-Nanoluc were used to treat human PBMC cells. Afterward, the supernatant was collected and analyzed by ELISA to measure levels of IFN-γ secreted by PBMCs under different treatment conditions. EVs loaded with NPTN-Ig1-3-TMD(NPTN-TMD)-ICD(EWI-F-ICD)-Nanoluc (scaffold control) were used as a control.

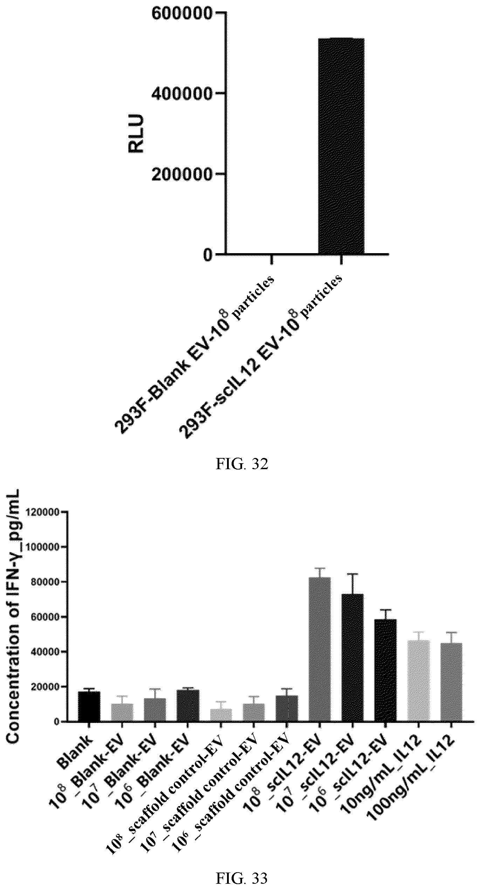

The results are shown in FIGs. 32-33.
1) Loading Verification: EVs loaded with scIL12 showed a high level of Nanoluc enzyme activity.
2) Efficacy Verification: EVs loaded with scIL12 can activate human PBMCs and stimulate its IFN-γ secretion compared to EVs loaded with the scaffold control in a significant dose-dependent manner. As the number of EVs particles increased, the activation of PBMC was gradually enhanced. 10⁶ EVs loaded with scIL-12 had a stronger activation effect than 10 ng/mL (2.4 ng) of scIL12 protein.

To sum up, by employing a modular design principle, the present disclosure selects and combines specific modules and optimizes screened mutant sequences to construct an improved nucleic acid construct for modular loading of a functional protein into engineered EVs, and finally obtains engineered EVs modularly loaded with functional protein. The product of the present disclosure has the following advantages: 1) achievement of modular loading of functional proteins in engineered EVs; 2) enhanced loading density and efficiency of proteins on and within the EV membranes by utilizing specific transmembrane domain sequences that synergistically interact with other modules; and 3) protection from enzyme cleavage by utilizing specific scaffold sequence. The nucleic acid construct obtained in the present disclosure enable the preparation of engineered EVs capable of modular loading of functional proteins, providing significant clinical application value and market prospect.

The nucleic acid construct for modular loading of a functional protein into engineered EVs and use thereof provided by the present disclosure are described in detail above. The principle and implementation of the present disclosure are illustrated by using specific embodiments herein. The above descriptions of the embodiments are only used to facilitate understanding of the method and the core idea of the present disclosure. It should be noted that, several improvements and modifications may be made by those skilled in the art to the present disclosure without departing from the principle of the present disclosure, and these improvements and modifications also fall within the protection scope of the claims of the present disclosure.

Sequence Listing

<?xml version="1.0" encoding="UTF-8"?>
<!DOCTYPE ST26SequenceListing SYSTEM "ST26SequenceListing_V1_3.dtd" PUBLIC "-
//WIPO//DTD Sequence Listing 1.3//EN">
<ST26SequenceListing productionDate="2023-05-18" softwareVersion="2.1.1"
softwareName="WIPO Sequence" fileName="OP230316.xml"
dtdVersion="V1_3"><ApplicantFileReference>OP230316</ApplicantFileReference><EarliestPr
iorityApplicationIdentification><IPOfficeCode>CN</IPOfficeCode><ApplicationNumberText>
202210549553.7</ApplicationNumberText><FilingDate>2022-05-
20</FilingDate></EarliestPriorityApplicationIdentification><ApplicantName
languageCode="zh">谛邈生物科技（北京）有限公司
</ApplicantName><ApplicantNameLatin>PANEXO BIOSCIENCES
C0.,LTD</ApplicantNameLatin><InventionTitle languageCode="zh"> 一种实现工程化 EVs
功能蛋白模块化装载的核酸构建体及其应用
</InventionTitle><SequenceTotalQuantity>27</SequenceTotalQuantity><SequenceData
sequenceIDNumber="1"><INSDSeq><INSDSeq_length>228</INSDSeq_length><INSDSeq_m
oltype>AA</INSDSeq_moltype><INSDSeq_division>PAT</INSDSeq_division><INSDSeq_fea
ture-
table><INSDFeature><INSDFeature_key>source</INSDFeature_key><INSDFeature_location>
1..228</INSDFeature_location><INSDFeature_quals><INSDQualifier><INSDQualifier_name>
mol_type</INSDQualifier_name><INSDQualifier_value>protein</INSDQualifier_value></INS
DQualifier><INSDQualifier
id="q28"><INSDQualifier_name>organism</INSDQualifier_name><INSDQualifier_value>synt
hetic
construct</INSDQualifier_value></INSDQualifier></INSDFeature_quals></INSDFeature></IN
SDSeq_feature-
table><INSDSeq_sequence>MPVKGGTKCIKYLLFGFNFIFWLAGIAVLAIGLWLRFDSQTK
SIFEQETNNNNNSSFYTGVYILIGAGALMMLVGFLGCCGAVQESQCMLGLFFGFLLVIFAIE
IAAAIWGYSHKDEVIKEVQEFYKDTYNKLKTKDEPQRETLKAIHYALNCCGLAGGVEQF
ISDICPKKDVLETFTVKSCPDAIKEVFDNKFHIIGAVGIGIAVVMIFGMIFSMILCCAIRRNR
EMV</INSDSeq_sequence></INSDSeq></SequenceData><SequenceData
sequenceIDNumber="2"><INSDSeq><INSDSeq_length>23</INSDSeq_length><INSDSeq_mol
type>AA</INSDSeq_moltype><INSDSeq_division>PAT</INSDSeq_division><INSDSeq_featu
re-
table><INSDFeature><INSDFeature_key>source</INSDFeature_key><INSDFeature_location>
1..23</INSDFeature_location><INSDFeature_quals><INSDQualifier><INSDQualifier_name>m
ol_type</INSDQualifier_name><INSDQualifier_value>protein</INSDQualifier_value></INSD
Qualifier><INSDQualifier
id="q29"><INSDQualifier_name>organism</INSDQualifier_name><INSDQualifier_value>synt
hetic
construct</INSDQualifier_value></INSDQualifier></INSDFeature_quals></INSDFeature></IN
SDSeq_feature-
table><INSDSeq_sequence>LLIGVGLSTVIGLLSCLIGYCSS</INSDSeq_sequence></INSDSe
q></SequenceData><SequenceData

sequenceIDNumber="3"><INSDSeq><INSDSeq_length>21</INSDSeq_length><INSDSeq_mol
type>AA</INSDSeq_moltype><INSDSeq_division>PAT</INSDSeq_division><INSDSeq_featu
re-
table><INSDFeature><INSDFeature_key>source</INSDFeature_key><INSDFeature_location>
1..21</INSDFeature_location><INSDFeature_quals><INSDQualifier><INSDQualifier_name>m
ol_type</INSDQualifier_name><INSDQualifier_value>protein</INSDQualifier_value></INSD
Qualifier><INSDQualifier
id="q30"><INSDQualifier_name>organism</INSDQualifier_name><INSDQualifier_value>synt
hetic
construct</INSDQualifier_value></INSDQualifier></INSDFeature_quals></INSDFeature></IN
SDSeq_feature-
table><INSDSeq_sequence>LFVPLLVGTGVALVTGATVLG</INSDSeq_sequence></INSDSe
q></SequenceData><SequenceData
sequenceIDNumber="4"><INSDSeq><INSDSeq_length>21</INSDSeq_length><INSDSeq_mol
type>AA</INSDSeq_moltype><INSDSeq_division>PAT</INSDSeq_division><INSDSeq_featu
re-
table><INSDFeature><INSDFeature_key>source</INSDFeature_key><INSDFeature_location>
1..21</INSDFeature_location><INSDFeature_quals><INSDQualifier><INSDQualifier_name>m
ol_type</INSDQualifier_name><INSDQualifier_value>protein</INSDQualifier_value></INSD
Qualifier><INSDQualifier
id="q31"><INSDQualifier_name>organism</INSDQualifier_name><INSDQualifier_value>synt
hetic
construct</INSDQualifier_value></INSDQualifier></INSDFeature_quals></INSDFeature></IN
SDSeq_feature-
table><INSDSeq_sequence>ALWPFLGIVAEVLVLVTIIFI</INSDSeq_sequence></INSDSeq>
</SequenceData><SequenceData
sequenceIDNumber="5"><INSDSeq><INSDSeq_length>23</INSDSeq_length><INSDSeq_mol
type>AA</INSDSeq_moltype><INSDSeq_division>PAT</INSDSeq_division><INSDSeq_featu
re-
table><INSDFeature><INSDFeature_key>source</INSDFeature_key><INSDFeature_location>
1..23</INSDFeature_location><INSDFeature_quals><INSDQualifier><INSDQualifier_name>m
ol_type</INSDQualifier_name><INSDQualifier_value>protein</INSDQualifier_value></INSD
Qualifier><INSDQualifier
id="q32"><INSDQualifier_name>organism</INSDQualifier_name><INSDQualifier_value>synt
hetic
construct</INSDQualifier_value></INSDQualifier></INSDFeature_quals></INSDFeature></IN
SDSeq_feature-
table><INSDSeq_sequence>LLIGVNLLTVIGLWLFLILYCSL</INSDSeq_sequence></INSDS
eq></SequenceData><SequenceData
sequenceIDNumber="6"><INSDSeq><INSDSeq_length>518</INSDSeq_length><INSDSeq_m
oltype>AA</INSDSeq_moltype><INSDSeq_division>PAT</INSDSeq_division><INSDSeq_fea
ture-
table><INSDFeature><INSDFeature_key>source</INSDFeature_key><INSDFeature_location>
1..518</INSDFeature_location><INSDFeature_quals><INSDQualifier><INSDQualifier_name>
mol_type</INSDQualifier_name><INSDQualifier_value>protein</INSDQualifier_value></INS
DQualifier><INSDQualifier

id="q33"><INSDQualifier_name>organism</INSDQualifier_name><INSDQualifier_value>synt
hetic
construct</INSDQualifier_value></INSDQualifier></INSDFeature_quals></INSDFeature></IN
SDSeq_feature-
table><INSDSeq_sequence>IWELKKDVYVVELDWYPDAPGEMVVLTCDTPEEDGITWTL
DQSSEVLGSGKTLTIQVKEFGDAGQYTCHKGGEVLSHSLLLLHKKEDGIWSTDILKDQK
EPKNKTFLRCEAKNYSGRFTCWWLTTISTDLTFSVKSSRGSSDPQGVTCGAATLSAERVR
GDNKEYEYSVECQEDSACPAAEESLPIEVMVDAVHKLKYENYTSSFFIRDIIKPDPPKNLQ
LKPLKNSRQVEVSWEYPDTWSTPHSYFSLTFCVQVQGKSKREKKDRVFTDKTSATVICR
KNASISVRAQDRYYSSSWSEWASVPCSGGGGSGGGGSGGGGSRNLPVATPDPGMFPCL
HHSQNLLRAVSNMLQKARQTLEFYPCTSEEIDHEDITKDKTSTVEACLPLELTKNESCLN
SRETSFITNGSCLASRKTSFMMALCLSSIYEDLKMYQVEFKTMNAKLLMDPKRQIFLDQN
MLAVIDELMQALNFNSETVPQKSSLEEPDFYKTKIKLCILLHAFRIRAVTIDRVMSYLNAS
</INSDSeq_sequence></INSDSeq></SequenceData><SequenceData
sequenceIDNumber="7"><INSDSeq><INSDSeq_length>167</INSDSeq_length><INSDSeq_m
oltype>AA</INSDSeq_moltype><INSDSeq_division>PAT</INSDSeq_division><INSDSeq_fea
ture-
table><INSDFeature><INSDFeature_key>source</INSDFeature_key><INSDFeature_location>
1..167</INSDFeature_location><INSDFeature_quals><INSDQualifier><INSDQualifier_name>
mol_type</INSDQualifier_name><INSDQualifier_value>protein</INSDQualifier_value></INS
DQualifier><INSDQualifier
id="q34"><INSDQualifier_name>organism</INSDQualifier_name><INSDQualifier_value>synt
hetic
construct</INSDQualifier_value></INSDQualifier></INSDFeature_quals></INSDFeature></IN
SDSeq_feature-
table><INSDSeq_sequence>VFTLEDFVGDWRQTAGYNLDQVLEQGGVSSLFQNLGVSVTP
IQRIVLSGENGLKIDIHVIIPYEGLSGDQMGQIEKIFKVVYPVDDHHFKVILHYGTLVIDGV
TPNMIDYFGRPYEGIAVFDGKKITVTGTLWNGNKIIDERLINPDGSLLFRVTINGVTGWRL
CER</INSDSeq_sequence></INSDSeq></SequenceData><SequenceData
sequenceIDNumber="8"><INSDSeq><INSDSeq_length>23</INSDSeq_length><INSDSeq_mol
type>AA</INSDSeq_moltype><INSDSeq_division>PAT</INSDSeq_division><INSDSeq_featu
re-
table><INSDFeature><INSDFeature_key>source</INSDFeature_key><INSDFeature_location>
1..23</INSDFeature_location><INSDFeature_quals><INSDQualifier><INSDQualifier_name>m
ol_type</INSDQualifier_name><INSDQualifier_value>protein</INSDQualifier_value></INSD
Qualifier><INSDQualifier
id="q35"><INSDQualifier_name>organism</INSDQualifier_name><INSDQualifier_value>synt
hetic
construct</INSDQualifier_value></INSDQualifier></INSDFeature_quals></INSDFeature></IN
SDSeq_feature-
table><INSDSeq_sequence>LLIGVGLLTVIGLLLFLILYCSS</INSDSeq_sequence></INSDSe
q></SequenceData><SequenceData
sequenceIDNumber="9"><INSDSeq><INSDSeq_length>23</INSDSeq_length><INSDSeq_mol
type>AA</INSDSeq_moltype><INSDSeq_division>PAT</INSDSeq_division><INSDSeq_featu
re-
table><INSDFeature><INSDFeature_key>source</INSDFeature_key><INSDFeature_location>

1..23</INSDFeature_location><INSDFeature_quals><INSDQualifier><INSDQualifier_name>m
ol_type</INSDQualifier_name><INSDQualifier_value>protein</INSDQualifier_value></INSD
Qualifier><INSDQualifier
id="q36"><INSDQualifier_name>organism</INSDQualifier_name><INSDQualifier_value>synt
hetic
construct</INSDQualifier_value></INSDQualifier></INSDFeature_quals></INSDFeature></IN
SDSeq_feature-
table><INSDSeq_sequence>LLIGVGLSTVIGLLSCLISYCSS</INSDSeq_sequence></INSDSe
q></SequenceData><SequenceData
sequenceIDNumber="10"><INSDSeq><INSDSeq_length>21</INSDSeq_length><INSDSeq_m
oltype>AA</INSDSeq_moltype><INSDSeq_division>PAT</INSDSeq_division><INSDSeq_fea
ture-
table><INSDFeature><INSDFeature_key>source</INSDFeature_key><INSDFeature_location>
1..21</INSDFeature_location><INSDFeature_quals><INSDQualifier><INSDQualifier_name>m
ol_type</INSDQualifier_name><INSDQualifier_value>protein</INSDQualifier_value></INSD
Qualifier><INSDQualifier
id="q37"><INSDQualifier_name>organism</INSDQualifier_name><INSDQualifier_value>synt
hetic
construct</INSDQualifier_value></INSDQualifier></INSDFeature_quals></INSDFeature></IN
SDSeq_feature-
table><INSDSeq_sequence>LWPFLGILAEIIILVVIIVVY</INSDSeq_sequence></INSDSeq><
/SequenceData><SequenceData
sequenceIDNumber="11"><INSDSeq><INSDSeq_length>239</INSDSeq_length><INSDSeq_
moltype>AA</INSDSeq_moltype><INSDSeq_division>PAT</INSDSeq_division><INSDSeq_f
eature-
table><INSDFeature><INSDFeature_key>source</INSDFeature_key><INSDFeature_location>
1..239</INSDFeature_location><INSDFeature_quals><INSDQualifier><INSDQualifier_name>
mol_type</INSDQualifier_name><INSDQualifier_value>protein</INSDQualifier_value></INS
DQualifier><INSDQualifier
id="q38"><INSDQualifier_name>organism</INSDQualifier_name><INSDQualifier_value>synt
hetic
construct</INSDQualifier_value></INSDQualifier></INSDFeature_quals></INSDFeature></IN
SDSeq_feature-
table><INSDSeq_sequence>MVSKGEELFTGVVPILVELDGDVNGHKFSVSGEGEGDATYG
KLTLKFICTTGKLPVPWPTLVTTLTYGVQCFSRYPDHMKQHDFFKSAMPEGYVQERTIFF
KDDGNYKTRAEVKFEGDTLVNRIELKGIDFKEDGNILGHKLEYNYNSHNVYIMADKQK
NGIKVNFKIRHNIEDGSVQLADHYQQNTPIGDGPVLLPDNHYLSTQSALSKDPNEKRDH
MVLLEFVTAAGITLGMDELYK</INSDSeq_sequence></INSDSeq></SequenceData><Seque
nceData
sequenceIDNumber="12"><INSDSeq><INSDSeq_length>229</INSDSeq_length><INSDSeq_
moltype>AA</INSDSeq_moltype><INSDSeq_division>PAT</INSDSeq_division><INSDSeq_f
eature-
table><INSDFeature><INSDFeature_key>source</INSDFeature_key><INSDFeature_location>
1..229</INSDFeature_location><INSDFeature_quals><INSDQualifier><INSDQualifier_name>
mol_type</INSDQualifier_name><INSDQualifier_value>protein</INSDQualifier_value></INS
DQualifier><INSDQualifier

id="q39"><INSDQualifier_name>organism</INSDQualifier_name><INSDQualifier_value>synt
hetic
construct</INSDQualifier_value></INSDQualifier></INSDFeature_quals></INSDFeature></IN
SDSeq_feature-
table><INSDSeq_sequence>AESKYGPPCPPCPAPEAAGGPSVFLFPPKPKDTLMISRTPEVT
CVVVDVSQEDPEVQFNWYVDGVEVHNAKTKPREEQFNSTYRVVSVLTVLHQDWLNGK
EYKCKVSNKGLPSSIEKTISKAKGQPREPQVYTLPPSQEEMTKNQVSLTCLVKGFYPSDIA
VEWESNGQPENNYKTTPPVLDSDGSFFLYSRLTVDKSRWQEGNVFSCSVMHEALHNHY
TQKSLSLSLG</INSDSeq_sequence></INSDSeq></SequenceData><SequenceData
sequenceIDNumber="13"><INSDSeq><INSDSeq_length>59</INSDSeq_length><INSDSeq_m
oltype>AA</INSDSeq_moltype><INSDSeq_division>PAT</INSDSeq_division><INSDSeq_fea
ture-
table><INSDFeature><INSDFeature_key>source</INSDFeature_key><INSDFeature_location>
1..59</INSDFeature_location><INSDFeature_quals><INSDQualifier><INSDQualifier_name>m
ol_type</INSDQualifier_name><INSDQualifier_value>protein</INSDQualifier_value></INSD
Qualifier><INSDQualifier
id="q40"><INSDQualifier_name>organism</INSDQualifier_name><INSDQualifier_value>synt
hetic
construct</INSDQualifier_value></INSDQualifier></INSDFeature_quals></INSDFeature></IN
SDSeq_feature-
table><INSDSeq_sequence>MGRAMVARLGLGLLLLALLLPTQIYSSETTTGTSSNSSQSTS
NSGLAPNPTNATTKAAG</INSDSeq_sequence></INSDSeq></SequenceData><SequenceDat
a
sequenceIDNumber="14"><INSDSeq><INSDSeq_length>310</INSDSeq_length><INSDSeq_
moltype>AA</INSDSeq_moltype><INSDSeq_division>PAT</INSDSeq_division><INSDSeq_f
eature-
table><INSDFeature><INSDFeature_key>source</INSDFeature_key><INSDFeature_location>
1..310</INSDFeature_location><INSDFeature_quals><INSDQualifier><INSDQualifier_name>
mol_type</INSDQualifier_name><INSDQualifier_value>protein</INSDQualifier_value></INS
DQualifier><INSDQualifier
id="q41"><INSDQualifier_name>organism</INSDQualifier_name><INSDQualifier_value>synt
hetic
construct</INSDQualifier_value></INSDQualifier></INSDFeature_quals></INSDFeature></IN
SDSeq_feature-
table><INSDSeq_sequence>ASKVYDPEQRKRMITGPQWWARCKQMNVLDSFINYYDSEK
HAENAVIFLHGNAASSYLWRHVVPHIEPVARCIIPDLIGMGKSGKSGNGSYRLLDHYKYL
TAWFELLNLPKKIIFVGHDWGACLAFHYSYEHQDKIKAIVHAESVVDVIESWDEWPDIEE
DIALIKSEEGEKMVLENNFFVETMLPSKIMRKLEPEEFAAYLEPFKEKGEVRRPTLSWPRE
IPLVKGGKPDVVQIVRNYNAYLRASDDLPKMFIESDPGFFSNAIVEGAKKFPNTEFVKVK
GLHFSQEDAPDEMGKYIKSFVERVLKNEQ</INSDSeq_sequence></INSDSeq></Sequence
Data><SequenceData
sequenceIDNumber="15"><INSDSeq><INSDSeq_length>515</INSDSeq_length><INSDSeq_
moltype>AA</INSDSeq_moltype><INSDSeq_division>PAT</INSDSeq_division><INSDSeq_f
eature-
table><INSDFeature><INSDFeature_key>source</INSDFeature_key><INSDFeature_location>
1..515</INSDFeature_location><INSDFeature_quals><INSDQualifier><INSDQualifier_name>

mol_type</INSDQualifier_name><INSDQualifier_value>protein</INSDQualifier_value></INSDQualifier><INSDQualifier
id="q42"><INSDQualifier_name>organism</INSDQualifier_name><INSDQualifier_value>synthetic
construct</INSDQualifier_value></INSDQualifier></INSDFeature_quals></INSDFeature></INSDSeq_feature-
table><INSDSeq_sequence>AESKYGPPCPPCPAPEAAGGPSVFLFPPKPKDTLMISRTPEVT
CVVVDVSQEDPEVQFNWYVDGVEVHNAKTKPREEQFNSTYRVVSVLTVLHQDWLNGK
EYKCKVSNKGLPSSIEKTISKAKGQPREPQVYTLPPSQEEMTKNQVSLTCLVKGFYPSDIA
VEWESNGQPENNYKTTPPVLDSDGSFFLYSRLTVDKSRWQEGNVFSCSVMHEALHNHY
TQKSLSLSLGQNAGFVKSPMSETKLTGDAFELYCDVVGSPTPEIQWWYAEVNRAESFRQ
LWDGARKRRVTVNTAYGSNGVSVLRITRLTLEDSGTYECRASNDPKRNDLRQNPSITPRI
VTSEEVIIRDSPVLPVTLQCNLTSSSHTLTYSYWTKNGVELSATRKNASNMEYRINKPRA
EDSGEYHCVYHFVSAPKANATIEVKPDITGHKRSENKNEGQDATMYCKSVGYPHPDWI
WRKKENGMPMDIVNTSGRFFIINKENYTELNIVNLQITEDPGEYECNATNAIGSASVVT</
INSDSeq_sequence></INSDSeq></SequenceData><SequenceData
sequenceIDNumber="16"><INSDSeq><INSDSeq_length>170</INSDSeq_length><INSDSeq_
moltype>AA</INSDSeq_moltype><INSDSeq_division>PAT</INSDSeq_division><INSDSeq_f
eature-
table><INSDFeature><INSDFeature_key>source</INSDFeature_key><INSDFeature_location>
1..170</INSDFeature_location><INSDFeature_quals><INSDQualifier><INSDQualifier_name>
mol_type</INSDQualifier_name><INSDQualifier_value>protein</INSDQualifier_value></INS
DQualifier><INSDQualifier
id="q43"><INSDQualifier_name>organism</INSDQualifier_name><INSDQualifier_value>synt
hetic
construct</INSDQualifier_value></INSDQualifier></INSDFeature_quals></INSDFeature></IN
SDSeq_feature-
table><INSDSeq_sequence>CQSEGIPESPKCHEGNGTFECGACRCNEGRVGRHCECSTDEV
NSEDMDAYCRKENSSEICSNNGECVCGQCVCRKRDNTNEIYSGKFCECDNFNCDRSNGL
ICGGNGVCKCRVCECNPNYTGSACDCSLDTSTCEASNGQICNGRGICECGVCKCTDPKF
QGQTCEMCQT</INSDSeq_sequence></INSDSeq></SequenceData><SequenceData
sequenceIDNumber="17"><INSDSeq><INSDSeq_length>313</INSDSeq_length><INSDSeq_
moltype>AA</INSDSeq_moltype><INSDSeq_division>PAT</INSDSeq_division><INSDSeq_f
eature-
table><INSDFeature><INSDFeature_key>source</INSDFeature_key><INSDFeature_location>
1..313</INSDFeature_location><INSDFeature_quals><INSDQualifier><INSDQualifier_name>
mol_type</INSDQualifier_name><INSDQualifier_value>protein</INSDQualifier_value></INS
DQualifier><INSDQualifier
id="q44"><INSDQualifier_name>organism</INSDQualifier_name><INSDQualifier_value>synt
hetic
construct</INSDQualifier_value></INSDQualifier></INSDFeature_quals></INSDFeature></IN
SDSeq_feature-
table><INSDSeq_sequence>YIPEAPRDGQAYVRKDGEWVLLSTFLGQNAGFVKSPMSETK
LTGDAFELYCDVVGSPTPEIQWWYAEVNRAESFRQLWDGARKRRVTVNTAYGSNGVS
VLRITRLTLEDSGTYECRASNDPKRNDLRQNPSITPRIVTSEEVIIRDSPVLPVTLQCNLTSS
SHTLTYSYWTKNGVELSATRKNASNMEYRINKPRAEDSGEYHCVYHFVSAPKANATIEV

KPDITGHKRSENKNEGQDATMYCKSVGYPHPDWIWRKKENGMPMDIVNTSGRFFIINKE
NYTELNIVNLQITEDPGEYECNATNAIGSASVVT</INSDSeq_sequence></INSDSeq></Seq
uenceData><SequenceData
sequenceIDNumber="18"><INSDSeq><INSDSeq_length>118</INSDSeq_length><INSDSeq_
moltype>AA</INSDSeq_moltype><INSDSeq_division>PAT</INSDSeq_division><INSDSeq_f
eature-
table><INSDFeature><INSDFeature_key>source</INSDFeature_key><INSDFeature_location>
1..118</INSDFeature_location><INSDFeature_quals><INSDQualifier><INSDQualifier_name>
mol_type</INSDQualifier_name><INSDQualifier_value>protein</INSDQualifier_value></INS
DQualifier><INSDQualifier
id="q45"><INSDQualifier_name>organism</INSDQualifier_name><INSDQualifier_value>synt
hetic
construct</INSDQualifier_value></INSDQualifier></INSDFeature_quals></INSDFeature></IN
SDSeq_feature-
table><INSDSeq_sequence>QLLFNKTKSVEFTFCNDTVVIPCFVTNMEAQNTTEVYVKWK
FKGRDIYTFDGALNKSTVPTDFSSAKIEVSQLLKGDASLKMDKSDAVSHTGNYTCEVTE
LTREGETIIELKYRVVSW</INSDSeq_sequence></INSDSeq></SequenceData><SequenceDat
a
sequenceIDNumber="19"><INSDSeq><INSDSeq_length>146</INSDSeq_length><INSDSeq_
moltype>AA</INSDSeq_moltype><INSDSeq_division>PAT</INSDSeq_division><INSDSeq_f
eature-
table><INSDFeature><INSDFeature_key>source</INSDFeature_key><INSDFeature_location>
1..146</INSDFeature_location><INSDFeature_quals><INSDQualifier><INSDQualifier_name>
mol_type</INSDQualifier_name><INSDQualifier_value>protein</INSDQualifier_value></INS
DQualifier><INSDQualifier
id="q46"><INSDQualifier_name>organism</INSDQualifier_name><INSDQualifier_value>synt
hetic
construct</INSDQualifier_value></INSDQualifier></INSDFeature_quals></INSDFeature></IN
SDSeq_feature-
table><INSDSeq_sequence>GDQNPQIAAHVISEASSKTTSVLQWAEKGYYTMSNNLVTLE
NGKQLTVKRQGLYYIYAQVTFCSNREASSQAPFIASLCLKSPGRFERILLRAANTHSSAKP
CGQQSIHLGGVFELQPGASVFVNVTDPSQVSHGTGFTSFGLLKL</INSDSeq_sequence></I
NSDSeq></SequenceData><SequenceData
sequenceIDNumber="20"><INSDSeq><INSDSeq_length>299</INSDSeq_length><INSDSeq_
moltype>AA</INSDSeq_moltype><INSDSeq_division>PAT</INSDSeq_division><INSDSeq_f
eature-
table><INSDFeature><INSDFeature_key>source</INSDFeature_key><INSDFeature_location>
1..299</INSDFeature_location><INSDFeature_quals><INSDQualifier><INSDQualifier_name>
mol_type</INSDQualifier_name><INSDQualifier_value>protein</INSDQualifier_value></INS
DQualifier><INSDQualifier
id="q47"><INSDQualifier_name>organism</INSDQualifier_name><INSDQualifier_value>synt
hetic
construct</INSDQualifier_value></INSDQualifier></INSDFeature_quals></INSDFeature></IN
SDSeq_feature-
table><INSDSeq_sequence>KVEQAVETEPEPELRQQTEWQSGQRWELALGRFWDYLRWV
QTLSEQVQEELLSSQVTQELRALMDETMKELKAYKSELEEQLTPVAEETRARLSKELQA

AQARLGADMEDVCGRLVQYRGEVQAMLGQSTEELRVRLASHLRKLRKRLLRDADDLQ
KRLAVYQAGAREGAERGLSAIRERLGPLVEQGRVRAATVGSLAGQPLQERAQAWGERL
RARMEEMGSRTRDRLDEVKEQVAEVRAKLEEQAQQIRLQAEAFQARLKSWFEPLVEDM
QRQWAGLVEKVQAAVGTSAAPVPSDNH</INSDSeq_sequence></INSDSeq></SequenceDa
ta><SequenceData
sequenceIDNumber="21"><INSDSeq><INSDSeq_length>249</INSDSeq_length><INSDSeq_
moltype>AA</INSDSeq_moltype><INSDSeq_division>PAT</INSDSeq_division><INSDSeq_f
eature-
table><INSDFeature><INSDFeature_key>source</INSDFeature_key><INSDFeature_location>
1..249</INSDFeature_location><INSDFeature_quals><INSDQualifier><INSDQualifier_name>
mol_type</INSDQualifier_name><INSDQualifier_value>protein</INSDQualifier_value></INS
DQualifier><INSDQualifier
id="q48"><INSDQualifier_name>organism</INSDQualifier_name><INSDQualifier_value>synt
hetic
construct</INSDQualifier_value></INSDQualifier></INSDFeature_quals></INSDFeature></IN
SDSeq_feature-
table><INSDSeq_sequence>QTVVTQEPSLTVSPGGTVTLTCGSSTGAVTSGNYPNWVQQK
PGQAPRGLIGGTKFLAPGTPARFSGSLLGGKAALTLSGVQPEDEAEYYCVLWYSNRWVF
GGGTKLTVLGGGGSGGGGSGGGGSEVQLVESGGGLVQPGGSLKLSCAASGFTFNKYAM
NWVRQAPGKGLEWVARIRSKYNNYATYYADSVKDRFTISRDDSKNTAYLQMNNLKTE
DTAVYYCVRHGNFGNSYISYWAYWGQGTLVTVSS</INSDSeq_sequence></INSDSeq></
SequenceData><SequenceData
sequenceIDNumber="22"><INSDSeq><INSDSeq_length>246</INSDSeq_length><INSDSeq_
moltype>AA</INSDSeq_moltype><INSDSeq_division>PAT</INSDSeq_division><INSDSeq_f
eature-
table><INSDFeature><INSDFeature_key>source</INSDFeature_key><INSDFeature_location>
1..246</INSDFeature_location><INSDFeature_quals><INSDQualifier><INSDQualifier_name>
mol_type</INSDQualifier_name><INSDQualifier_value>protein</INSDQualifier_value></INS
DQualifier><INSDQualifier
id="q49"><INSDQualifier_name>organism</INSDQualifier_name><INSDQualifier_value>synt
hetic
construct</INSDQualifier_value></INSDQualifier></INSDFeature_quals></INSDFeature></IN
SDSeq_feature-
table><INSDSeq_sequence>DIQMTQSPSSLSASVGDRVTITCKSSQSLFNTGNQKNYLTWY
QQKPGKAPKLLIYWASTRESGVPSRFSGSGSGTDFTLTISSLQPEDFATYYCQNNYNFPLT
FGQGTRLEIKGGGGSGGGGSGGGGSQVQLVESGGGLVKPGGSLRLSCAASGFSFSSFGM
HWVRQAPGKGLEWVAYISSGSRTIYYADTVKGRFTISRDNAKNSLYLQMNSLRAEDTA
VYYCTRYYYGNSFDYWGQGTTVTVSS</INSDSeq_sequence></INSDSeq></SequenceData
><SequenceData
sequenceIDNumber="23"><INSDSeq><INSDSeq_length>242</INSDSeq_length><INSDSeq_
moltype>AA</INSDSeq_moltype><INSDSeq_division>PAT</INSDSeq_division><INSDSeq_f
eature-
table><INSDFeature><INSDFeature_key>source</INSDFeature_key><INSDFeature_location>
1..242</INSDFeature_location><INSDFeature_quals><INSDQualifier><INSDQualifier_name>
mol_type</INSDQualifier_name><INSDQualifier_value>protein</INSDQualifier_value></INS
DQualifier><INSDQualifier

id="q50"><INSDQualifier_name>organism</INSDQualifier_name><INSDQualifier_value>synt
hetic
construct</INSDQualifier_value></INSDQualifier></INSDFeature_quals></INSDFeature></IN
SDSeq_feature-
table><INSDSeq_sequence>DVVMTQSPLSLPVTPGEPASISCRSSQSLVHSNANTYLHWYL
QKPGQSPQLLIYKVSNRFSGVPDRFSGSGSGTDFTLKISRVEAEDVGVYYCSQNTHVPPT
FGQGTKLEIKGGGGSGGGGSGGGGSQVQLVQSGAEVKKPGASVKVSCKASGYTFTDYE
MHWVRQAPGQGLEWMGALDPKTGDTAYSQKFKGRVTLTADESTSTAYMELSSLRSED
TAVYYCTRFYSYTYWGQGTLVTVSS</INSDSeq_sequence></INSDSeq></SequenceData><
SequenceData
sequenceIDNumber="24"><INSDSeq><INSDSeq_length>211</INSDSeq_length><INSDSeq_
moltype>AA</INSDSeq_moltype><INSDSeq_division>PAT</INSDSeq_division><INSDSeq_f
eature-
table><INSDFeature><INSDFeature_key>source</INSDFeature_key><INSDFeature_location>
1..211</INSDFeature_location><INSDFeature_quals><INSDQualifier><INSDQualifier_name>
mol_type</INSDQualifier_name><INSDQualifier_value>protein</INSDQualifier_value></INS
DQualifier><INSDQualifier
id="q51"><INSDQualifier_name>organism</INSDQualifier_name><INSDQualifier_value>synt
hetic
construct</INSDQualifier_value></INSDQualifier></INSDFeature_quals></INSDFeature></IN
SDSeq_feature-
table><INSDSeq_sequence>MKLWDVVAVCLVLLHTASAFPLPAGKRPPEAPAEDRSLGRR
RAPFALSSDSNMPEDYPDQFDDVMDFIQATIKRLKRSPDKQMAVLPRRERNRQAAAANP
ENSRGKGRRGQRGKNRGCVLTAIHLNVTDLGLGYETKEELIFRYCSGSCDAAETTYDKI
LKNLSRNRRLVSDKVGQACCRPIAFDDDLSFLDDNLVYHILRKHSAKRCGCI</INSDSeq_
sequence></INSDSeq></SequenceData><SequenceData
sequenceIDNumber="25"><INSDSeq><INSDSeq_length>24</INSDSeq_length><INSDSeq_m
oltype>AA</INSDSeq_moltype><INSDSeq_division>PAT</INSDSeq_division><INSDSeq_fea
ture-
table><INSDFeature><INSDFeature_key>source</INSDFeature_key><INSDFeature_location>
1..24</INSDFeature_location><INSDFeature_quals><INSDQualifier><INSDQualifier_name>m
ol_type</INSDQualifier_name><INSDQualifier_value>protein</INSDQualifier_value></INSD
Qualifier><INSDQualifier
id="q52"><INSDQualifier_name>organism</INSDQualifier_name><INSDQualifier_value>synt
hetic
construct</INSDQualifier_value></INSDQualifier></INSDFeature_quals></INSDFeature></IN
SDSeq_feature-
table><INSDSeq_sequence>HWCCKKEVQETRRERRRLMSMEMD</INSDSeq_sequence></I
NSDSeq></SequenceData><SequenceData
sequenceIDNumber="26"><INSDSeq><INSDSeq_length>13</INSDSeq_length><INSDSeq_m
oltype>AA</INSDSeq_moltype><INSDSeq_division>PAT</INSDSeq_division><INSDSeq_fea
ture-
table><INSDFeature><INSDFeature_key>source</INSDFeature_key><INSDFeature_location>
1..13</INSDFeature_location><INSDFeature_quals><INSDQualifier><INSDQualifier_name>m
ol_type</INSDQualifier_name><INSDQualifier_value>protein</INSDQualifier_value></INSD
Qualifier><INSDQualifier

id="q53"><INSDQualifier_name>organism</INSDQualifier_name><INSDQualifier_value>synt
hetic
construct</INSDQualifier_value></INSDQualifier></INSDFeature_quals></INSDFeature></IN
SDSeq_feature-
table><INSDSeq_sequence>TITCCFMKRLRKR</INSDSeq_sequence></INSDSeq></Sequenc
eData><SequenceData
sequenceIDNumber="27"><INSDSeq><INSDSeq_length>23</INSDSeq_length><INSDSeq_m
oltype>AA</INSDSeq_moltype><INSDSeq_division>PAT</INSDSeq_division><INSDSeq_fea
ture-
table><INSDFeature><INSDFeature_key>source</INSDFeature_key><INSDFeature_location>
1..23</INSDFeature_location><INSDFeature_quals><INSDQualifier><INSDQualifier_name>m
ol_type</INSDQualifier_name><INSDQualifier_value>protein</INSDQualifier_value></INSD
Qualifier><INSDQualifier
id="q54"><INSDQualifier_name>organism</INSDQualifier_name><INSDQualifier_value>synt
hetic
construct</INSDQualifier_value></INSDQualifier></INSDFeature_quals></INSDFeature></IN
SDSeq_feature-
table><INSDSeq_sequence>IIPIVAGVVAGIVLIGLALLLIW</INSDSeq_sequence></INSDSe
q></SequenceData></ST26SequenceListing>

## Claims

1. A nucleic acid construct for modular loading of a functional protein into engineered EVs, comprising:
an effector module (Head), a scaffold module (Scaffold), a transmembrane domain module (TMD), and an intracellular domain anchoring module (ICD);
wherein the effector module, the scaffold module, the transmembrane domain module and the intracellular domain anchoring module are sequentially connected from 5' end to 3' end either directly or through a linking module, and the linking module comprises a linker peptide (Linker).

2. The nucleic acid construct according to claim 1, wherein the nucleic acid construct further comprises a detection module (Tail), wherein the 5' end of the detection module is connected to the 3' end of the intracellular domain anchoring module.

3. The nucleic acid construct according to claim 1 or 2, wherein the effector module comprises any one or more selected from the group consisting of scIL12, CD47, APOE, hCD24, ScFv, GDNF, and CD40L.

4. The nucleic acid construct according to any one of claims 1 to 3, wherein the scaffold module comprises any one or more selected from the group consisting of Fc, Foldon, Fc+Ig1 to Ig3 domains of NPTN (NPTN-Ig1-3), I-EGF1 to I-EGF4 domains of ITGB1 (ITGB1-I-EGF1-4), Foldon+NPTN-Ig1-3, and NPTN-Ig1-3.

5. The nucleic acid construct according to any one of claims 1 to 4, wherein the transmembrane domain module comprises any one or more selected from the group consisting of TMD of EWI-F (EWI-F-TMD), TMD of EWI-2 (EWI-2-TMD), TMD of ITGB1 (ITGB1-TMD), TMD_Mut_10, TMD_Mut_13, TMD_Mut_17, and TMD of NPTN (NPTN-TMD).

6. The nucleic acid construct according to any one of claims 1 to 5, wherein the transmembrane domain module comprises any one or more selected from the group consisting of TMD_Mut_10, TMD_Mut_13, TMD_Mut_17, and NPTN-TMD;
wherein TMD_Mut_10 has a nucleotide sequence that encodes an amino acid sequence set forth in SEQ ID NO. 5,
TMD_Mut_13 has a nucleotide sequence that encodes an amino acid sequence set forth in SEQ ID NO. 8,
TMD_Mut_17 has a nucleotide sequence that encodes an amino acid sequence set forth in SEQ ID NO. 9, and
NPTN-TMD has a nucleotide sequence that encodes an amino acid sequence set forth in SEQ ID NO. 10.

7. The nucleic acid construct according to any one of claims 1 to 6, wherein the intracellular domain anchoring module comprises a nucleotide sequence encoding S-palmitoyl cysteine, and the intracellular domain anchoring module comprises EWI-F-ICD or EWI-2-ICD.

8. The nucleic acid construct according to any one of claims 1 to 7, wherein the detection module comprises any one or more of a fluorescent protein module and/or a chemiluminescent module.

9. The nucleic acid construct according to claim 8, wherein the fluorescent protein module comprises any one or more selected from the group consisting of green fluorescent protein, red fluorescent protein, blue fluorescent protein, and yellow fluorescent protein; and the chemiluminescent module comprises nanoLuciferase (Nanoluc) or RLuciferase (Rluc).

10. A transmembrane domain mutant protein, comprising any one selected from the group consisting of TMD_Mut_10, TMD_Mut_13, and TMD_Mut_17;
wherein TMD_Mut_10 has a nucleotide sequence that encodes an amino acid sequence set forth in SEQ ID NO. 5,
TMD_Mut_13 has an amino acid sequence set forth in SEQ ID NO. 8, and
TMD_Mut_17 has an amino acid sequence set forth in SEQ ID NO. 9.

11. A nucleic acid construct comprising a nucleic acid molecule encoding the transmembrane domain mutant protein according to claim 10.

12. The nucleic acid construct according to claim 11, wherein the nucleic acid construct further comprises an effector module (Head), a scaffold module (Scaffold), a transmembrane domain module (TMD), and an intracellular domain anchoring module (ICD);
wherein the effector module, the scaffold module, the transmembrane domain module and the intracellular domain anchoring module are sequentially connected from 5' end to 3' end either directly or through a linking module, and the linking module comprises a linker peptide (Linker).

13. The nucleic acid construct according to claim 12, wherein the nucleic acid construct further comprises a detection module (Tail), wherein the 5' end of the detection module is connected to the 3' end of the intracellular domain anchoring module.

14. A plasmid, expression vector, or expression cassette, comprising the nucleic acid construct according to any one of claims 1 to 9 and 11 to 13.

15. A transformant or recombinant cell, comprising the plasmid, expression vector or expression cassette according to claim 14.

16. An engineered EV prepared using the transformant or recombinant cell according to claim 15.

17. A protein expressed by the nucleic acid construct according to claims 1 to 9 and 11 to 13.

18. Use of the nucleic acid construct according to any one of claims1 to 9 and 11 to 13, the plasmid, expression vector, or expression cassette according to claim 14, the transformant or recombinant cell according to claim 15, the engineered EV according to claim 16, and/or the protein according to claim 17 in the manufacture of a medicament or an agent, wherein the medicament has the effector module as an active ingredient.
